# EUROPEAN PATENT SPECIFICATION

(11) **EP 2 113 211 B1**
(45) Date of publication and mention of the grant of the patent: **01.12.2010**
(21) Application number: 09004504.8
(22) Date of filing: 27.03.2009
(51) Int. Cl.: A61B 17/32, A61B 17/29

(54) **Surgical operating apparatus**
Chirurgische Operationsvorrichtung
Appareil pour opération chirurgicale

(30) Priority: 28.04.2008 US 110922
(43) Date of publication of application: 04.11.2009
(73) Proprietor: Olympus Medical Systems Corp., Tokyo 151-0072 (JP)
(72) Inventor: Yachi, Chie, Hachioji-shi Tokyo 192-8512 (JP); Masuda, Shinya, Hachioji-shi Tokyo 192-8512 (JP)
(74) Representative: von Hellfeld, Axel

(56) References cited:
- US-A1- 2006 241 532
- US-B1- 6 458 142
- US-B1- 6 669 690

## Description

The present invention relates to a surgical operating apparatus by which a treatment such as incision, resection, and coagulation is executed by transmitting at least one of ultrasonic vibration and high-frequency waves to the probe distal end section in a state where a living tissue is grasped between a probe distal end section arranged at a distal end part of a vibration transmission member for transmitting ultrasonic vibration and a jaw supported so as to be openable/closable with respect to the probe distal end section.

In general, as an example of an ultrasonic treatment apparatus for executing a treatment such as incision, resection, and coagulation by utilizing ultrasonic waves, an ultrasonic treatment instrument described in, for example, Jpn. Pat. Appln. KOKAI Publication No. 2003-111770 (Pat. Document 1), and Jpn. Pat. Appln. KOKAI Publication No. 2003-265496 (Pat. Document 2) is disclosed.

In this apparatus, a operation section on the hand side is coupled to a proximal end part of an elongated insertion section. This operation section is provided with an ultrasonic transducer for generating ultrasonic vibration. A distal end part of the insertion section is provided with a treatment section for treating a living tissue.

The insertion section includes an elongated sheath having a tubular shape. A stick-like vibration transmission member (probe) is inserted into the sheath. The proximal end part of the vibration transmission member is detachably connected to the ultrasonic transducer through a screwed type coupling section. Further, ultrasonic vibration generated by the ultrasonic transducer is transmitted to the probe distal end section on the distal end side of the vibration transmission member.

The treatment section is provided with a jaw so as to be opposed to the probe distal end section. The proximal end part of the jaw is pivotally supported at a distal end part of the sheath through a support shaft. A drive shaft for driving the jaw is inserted into the sheath so that it can be advanced or retreated in the axial direction. A jaw main body is coupled to a distal end part of the drive shaft through a coupling pin.

Further, the operation section is provided with a operation handle used for the opening/closing operation of the jaw, a power transmission mechanism for converting the operation of the operation handle into the advancing/retreating movement of the drive shaft in the axial direction, and a rotary operation knob for rotating the entire insertion section with respect to the operation section in the direction around the axis. The operation handle includes a fixed handle and a movable handle that can be opened/closed with respect to the fixed handle. The power transmission mechanism includes a slider section which is advanced/retreated in the central axis direction of the vibration transmission member in accordance with the operation of the movable handle. A proximal end part of the drive shaft is fixed to a distal end of the slider section.

The slider section is formed of a cylindrical body. A ring-shaped groove is provided in the circumferential direction in the outer circumferential surface of the cylindrical body of the slider section. An action pin fixed to the movable handle is engaged with the ring-shaped groove in a state where the pin is inserted in the groove.

Further, when the movable handle is operated, the action pin in the ring-shaped groove of the slider section presses the side-wall surface of the groove. At this time, the drive shaft is driven to be advanced/retreated in the axial direction by the pressing force from the action pin through the slider section. The jaw is operated to be opened/closed with respect to the probe distal end section interlocking with the operation of the drive shaft.

Further, the living tissue is grasped between the probe distal end section and the jaw concomitantly with the closing operation of the jaw. In this state, the ultrasonic vibration from the ultrasonic transducer is transmitted to the probe distal end section of the treatment section side through the vibration transmission member, whereby the treatment such as incision, resection, and coagulation of the living tissue is executed by utilizing the ultrasonic waves.

Further, when the rotary operation knob is operated to be rotated, the entirety of the insertion section is rotated in the direction around the axis with respect to the operation section. At this time, the slider section is rotated in the direction around the axis together with the rotary operation knob. Accordingly, the slider section is slid in the circumferential direction along the ring-shaped groove in a state where the slider section is guided by the action pin of the movable handle.

Incidentally, a spring member such as a coil spring or the like acting in a direction in which the spring member resists the movement of the slider section is provided in the operation section at a certain equipped force amount. Further, at the time of the operation of the movable handle, when the slider section is driven to be advanced/retreated in the axial direction, if the handle operation force exceeds the equipped force amount of the spring member, the spring member is compressed, whereby excessive handle operation force is prevented from being transmitted to the drive shaft side through the slider section.

In the apparatus of the conventional configuration described above, when the movable handle is in an opened position with respect to the fixed hand, the rotary operation knob can be operated to be rotated by slight force. However, when the movable handle is operated in a direction in which the movable handle is closed with respect to the fixed handle such that the jaw is operated to be closed, and the living tissue is grasped between the probe distal end section and the jaw, large pressing force is generated between the action pin of the movable handle and the groove side-wall surface of the ring-shaped groove of the slider section. Thus, when the rotary operation knob is operated to be rotated in this state, the operation force becomes heavy and the operation is hard to perform. US 2006/0241532 discloses a surgical operating apparatus according to the preamble of claim 1.

The present invention has been contrived paying attention to these circumstances, and an object thereof is to provide a surgical operating apparatus having a rotary operation knob of which can be operated to be rotated by slight force even when a movable handle is operated in a direction in which the movable handle is closed with respect to a fixed handle, and which can prevent the load of the operation for rotating the rotary operation knob from becoming heavy, and enabling a stable operation to be performed.

According to an aspect of the present invention, there is provided a surgical operating apparatus comprising: a sheath provided with a distal end part and a proximal end part; an apparatus main body to be coupled to the proximal end part of the sheath; a probe which is inserted into the sheath, and transmits ultrasonic waves; a probe distal end section provided at a distal end part of the probe; and a jaw pivotally supported at the distal end part of the sheath, the jaw being supported so that the jaw can be operated to be opened or closed between a closed position at which the jaw is engaged with the probe distal end section, and an opened position at which the jaw is separated from the probe distal end section, wherein the apparatus main body includes: a handle for operating the opening/closing operation of the jaw; a slider section which is moved to be advanced/retreated in an axial direction of a central axis line of the probe in accordance with the operation of the handle; a pin which is provided to the handle, is engaged with the slider section, and transmits the operation of the handle to the slider section; and a frictional force reduction section which is provided to the slider section and/or the pin at a surface at which the slider section and the pin are engaged with each other, and reduces the frictional force acting at the engagement surface at which the slider section and the pin are engaged with each other.

Preferably, the frictional force reduction section is a resin member with low frictional properties.

Preferably, the resin member with low frictional properties is formed of a PTFE (polytetrafluoroethylene) resin material.

Preferably, the frictional force reduction section is a ring-shaped contact member which is provided at the engagement surface of the slider section at which the slider section and the pin are engaged with each other, and is formed of the resin member with low frictional properties.

Preferably, the slider section is provided with a fixing projection for fixing the contact member to the engagement surface of the slider section at which the slider section and the pin are engaged with each other.

Preferably, the frictional force reduction section is provided with a metallic washer for preventing the elasticity of the resin material of the contact member from being fatigued at an engagement surface at which the contact member and the pin are engaged with each other.

Preferably, the frictional force reduction section is a coating section which is provided at the engagement surface of the slider section at which the slider section and the pin are engaged with each other, and is formed by coating the surface of a metallic washer with the resin member with low frictional properties.

Preferably, the frictional force reduction section is a slider section formed of the resin material with low frictional properties.

Preferably, the frictional force reduction section is a pin formed of the resin material with low frictional properties.

Preferably, the frictional force reduction section is a coating section formed by coating the surface of the metallic pin with the resin material with low frictional properties.

The invention can be more fully understood from the following detailed description when taken in conjunction with the accompanying drawings, in which:
FIG. 1 is a perspective view showing a schematic configuration of an entire hand piece of a surgical operating apparatus according to a first embodiment of a present invention.
FIG. 2 is a perspective view showing a disassembled state in which a coupling part of an assembly unit of a hand piece of the surgical operating apparatus according to the first embodiment is detached.
FIG. 3 is a perspective view showing an external appearance of a handle unit of the surgical operating apparatus according to the first embodiment.
FIG. 4A is a longitudinal cross-sectional view showing a coupling state of the handle unit and a transducer unit of the surgical operating apparatus according to the first embodiment.
FIG. 4B is a cross-sectional view taken along line L4B-L4B of FIG. 4A.
FIG. 5A is a plan view showing a probe unit of the surgical operating apparatus according to the first embodiment.
FIG. 5B is a cross-sectional view taken along line L5B-L5B of FIG. 5A.
FIG. 6 is a longitudinal cross-sectional view of a sheath unit of the surgical operating apparatus according to the first embodiment.
FIG. 7 is a longitudinal cross-sectional view showing a coupling state of a jaw and a drive pipe of the surgical operating apparatus according to the first embodiment.
FIG. 8 is a cross-sectional view taken along line L8-L8 of FIG. 7.
FIG. 9 is a plan view showing a surface of the jaw of the surgical operating apparatus according to the first embodiment at which the jaw is opposed to a probe distal end section.
FIG. 10 is a transverse cross-sectional view taken at a cross-sectional position of line L10-L10 showing a state where a part between the jaw and the probe of the surgical operating apparatus according to the first embodiment is closed.
FIG. 11 is a longitudinal cross-sectional view slowing a proximal end part of the sheath unit of the surgical operating apparatus according to the first embodiment.
FIG. 12 is a cross-sectional view taken along line L12-L12 of FIG. 11.
FIG. 13 is a cross-sectional view taken along line L13-L13 of FIG. 11.
FIG. 14 is a longitudinal cross-sectional view showing a knob member of the surgical operating apparatus according to the first embodiment in a state before assembling.
FIG. 15 is a longitudinal cross-sectional view showing a guide cylindrical body of the sheath unit of the surgical operating apparatus according to the first embodiment.
FIG. 16 is a front view showing a proximal end part of the guide cylindrical body of the sheath unit of the surgical operating apparatus according to the first embodiment.
FIG. 17 is a longitudinal cross-sectional view showing a main part of a fixed handle of the surgical operating apparatus according to the first embodiment in a state before assembling.
FIG. 18 is a longitudinal cross-sectional view showing an internal structure of the handle unit of the surgical operating apparatus according to the first embodiment.
FIG. 19 is a cross-sectional view taken along line L19-L19 of FIG. 18.
FIG. 20 is a cross-sectional view taken along line L20-L20 of FIG. 18.
FIG. 21 is a cross-sectional view taken along line L21-L21 of FIG. 18.
FIG. 22A is a longitudinal cross-sectional view showing the handle unit and the sheath unit of the surgical operating apparatus according to the first embodiment in a state before engagement.
FIG. 22B is a longitudinal cross-sectional view showing the handle unit and the sheath unit of the surgical operating apparatus according to the first embodiment in a state after engagement.
FIG. 23 is a plan view of a main part showing a notification mechanism of the hand piece of the surgical operating apparatus according to the first embodiment.
FIG. 24 is a perspective view showing a guide cylindrical body of the notification mechanism of the surgical operating apparatus according to the first embodiment.
FIG. 25 is a perspective view showing a leaf spring member of the notification mechanism of the surgical operating apparatus according to the first embodiment.
FIG. 26 is a longitudinal cross-sectional view of a main part for explaining a movement of the leaf spring member of the notification mechanism of the surgical operating apparatus according to the first embodiment.
FIG. 27 is an explanatory view for explaining a deformed state of the coil spring observed when a slider member of the surgical operating apparatus according to the first embodiment is operated.
FIG. 28 is a longitudinal cross-sectional view of a main part showing a contact part at which a contact member of an engagement groove of the slider member and an action pin of a movable handle of the surgical operating apparatus according to the first embodiment are in contact with each other.
FIG. 29 is a longitudinal cross-sectional view of a main part showing a contact part at which an engagement groove of a slider member and an action pin of a movable handle of a surgical operating apparatus according to a second embodiment of the present invention are in contact with each other.
FIG. 30 is a longitudinal cross-sectional view of a main part showing a contact part at which an engagement groove of a slider member and an action pin of a movable handle of a surgical operating apparatus according to a third embodiment of the present invention are in contact with each other.
FIG. 31 is a longitudinal cross-sectional view of a main part showing a contact part at which an engagement groove of a slider member and an action pin of a movable handle of a surgical operating apparatus according to a fourth embodiment of the present invention are in contact with each other.
FIG. 32 is a transverse cross-sectional view of a main part showing a contact part at which an engagement groove of a slider member and an action pin of a movable handle of a surgical operating apparatus according to a fifth embodiment of the present invention are in contact with each other.
FIG. 33 is a longitudinal cross-sectional view of a main part showing the action pin of the movable handle of the surgical operating apparatus according to the fifth embodiment.

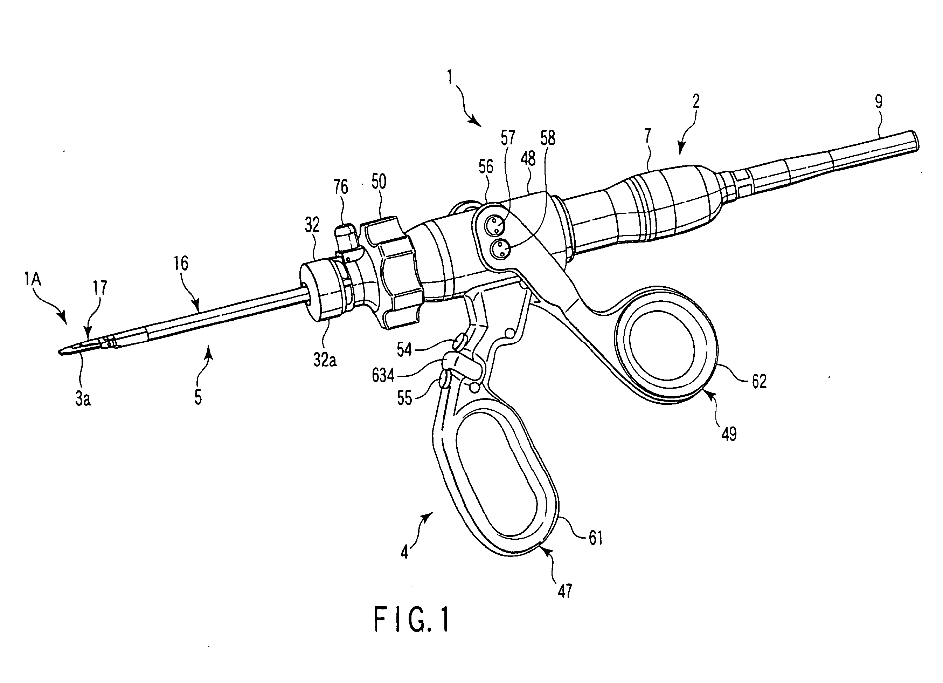

A first embodiment of the present invention will be described below with reference to FIGS. 1 to 28. FIG. 1 shows a schematic configuration of an entire hand piece 1 of an ultrasonic treatment apparatus which is a surgical operating apparatus of the first embodiment. The ultrasonic treatment apparatus of this embodiment is an ultrasonic coagulation-incision treatment apparatus. This ultrasonic coagulation-incision treatment apparatus can perform a treatment such as incision, resection, or coagulation of a living tissue by utilizing ultrasonic waves, as well as a treatment utilizing high-frequency waves.

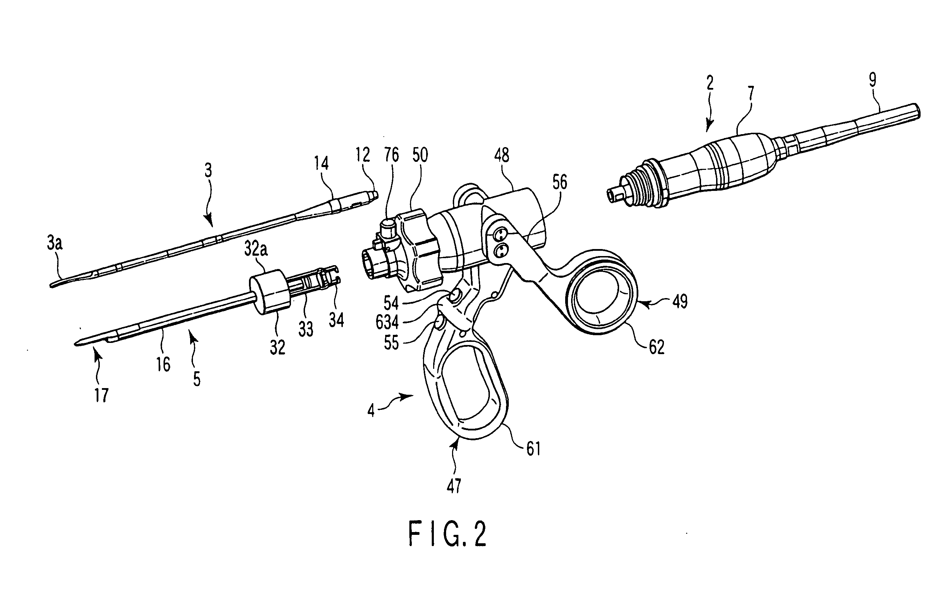

A hand piece 1 includes, as shown in FIG. 2, four nits of a transducer unit 2, a probe unit (probe section) 3, a handle unit (operation section) 4, and a sheath unit (sheath section) 5. These four units are coupled to each other so that they can be disassembled.

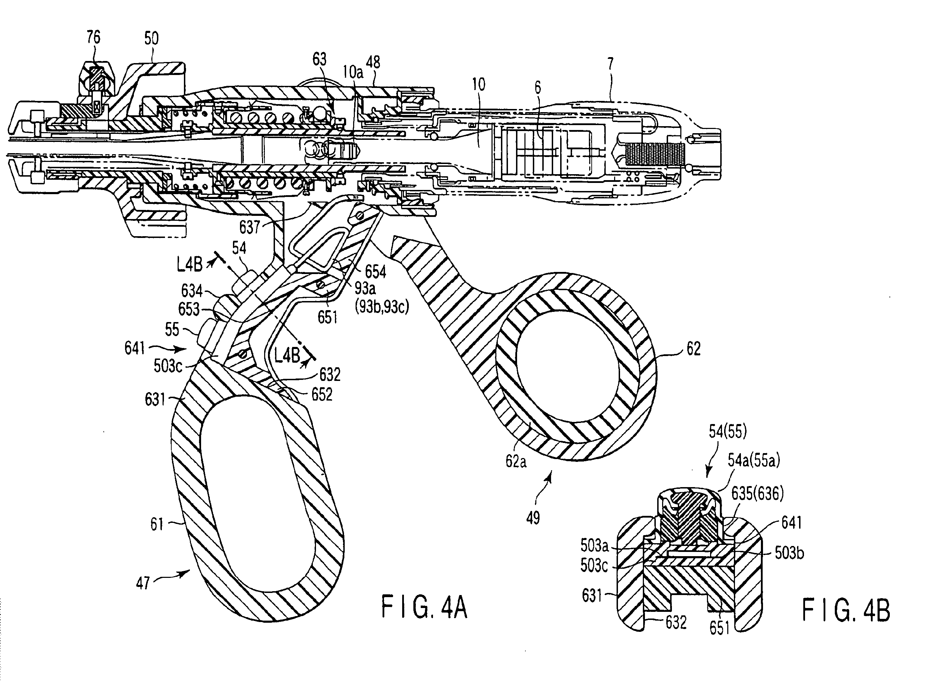

As shown in FIG. 4, an ultrasonic transducer 6 for generating an ultrasonic vibration by means of a piezoelectric element for converting a current into an ultrasonic vibration is incorporated in the transducer unit 2. The outside of the ultrasonic transducer 6 is covered with a cylindrical transducer cover 7. As shown in FIG. 1, a cable 9 for supplying a current for generating an ultrasonic vibration from a power source apparatus main body (not shown) is extended from a rear end of the transducer unit 2.

A proximal end part of a horn 10 for performing amplitude extension of the ultrasonic vibration is coupled to a front end part of the ultrasonic transducer 6. A threaded hole section 10a for attaching a probe is formed in a distal end part of the horn 10.

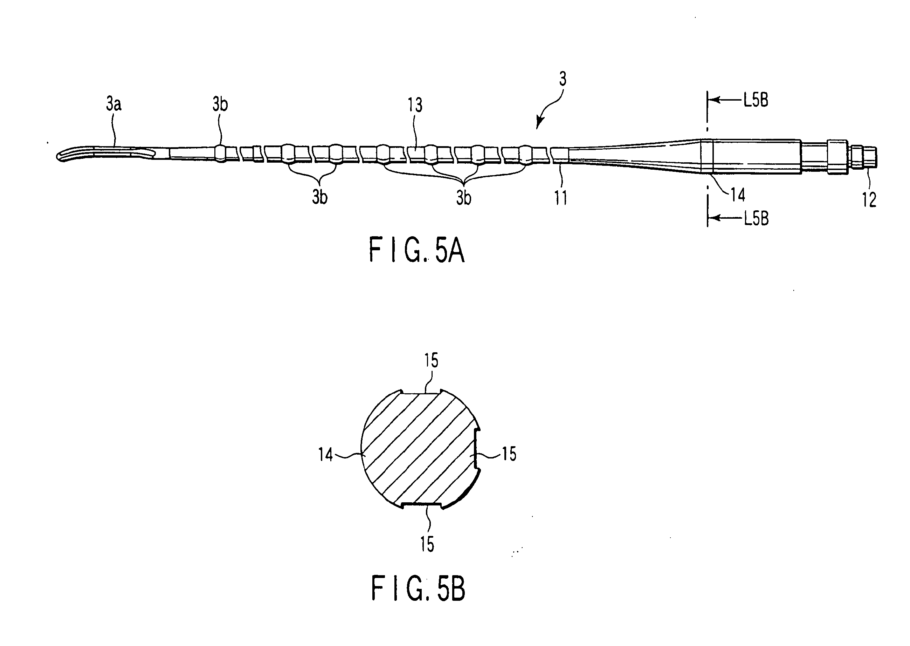

FIG. 5A shows an external appearance of the entire probe unit 3. This probe unit 3 is designed in such a manner that an overall length of the probe unit 3 is an integral multiple of a half wavelength of the ultrasonic vibration. The probe unit 3 includes a vibration transmission member 11 having a distal end part and a proximal end part, and a longitudinal axis, made of metal, and having a stick-like shape. A screw section 12 to be screwed into the threaded hole section 10a of the horn 10 is provided at a proximal end part of the vibration transmission member 11. Further, the screw section 12 is screwed into the threaded hole section 10a of the horn 10 of the transducer unit 2 so as to be attached thereto. As a result of this, the probe unit 3 and the transducer unit 2 are coupled to each other. At this time, in the connected body of the ultrasonic transducer 6 and the probe unit 3, a first high-frequency electric pathway 13 through which a high-frequency current is transmitted is formed.

A probe distal end section 3a is provided at the distal end part of the vibration transmission member 11. The probe distal end section 3a is formed into a curved shape of a substantially J-shape. Further, the probe distal end section 3a constitutes a first electrode section which is one of bipolar electrodes. In the probe unit 3, the cross-sectional area is reduced at several node positions of the vibration on the midway in the axial direction so that an amplitude necessary for the treatment can be obtained at the probe distal end section 3a. Rubber rings 3b formed of an elastic member with a ring-like shape are attached to the probe unit 3 at several node positions of the vibration on the midway in the axial direction of the probe unit 3. Further, these rubber rings 3b prevent interference between the probe unit 3 and the sheath unit 5 from occurring.

A flange section 14 is provided at the closest node position of the vibration to the proximal end side in the axial direction of the probe unit 3. An oddly shaped part having a noncircular shape for preventing a heterogeneous type from being attached is formed on the outer circumferential surface of the flange section 14. This oddly shaped part has a shape obtained by forming, for example, engagement concave sections 15 having a keyway-shape at three positions on the outer circumferential surface of the flange section 14 in the circumferential direction as shown in FIG. 5B.

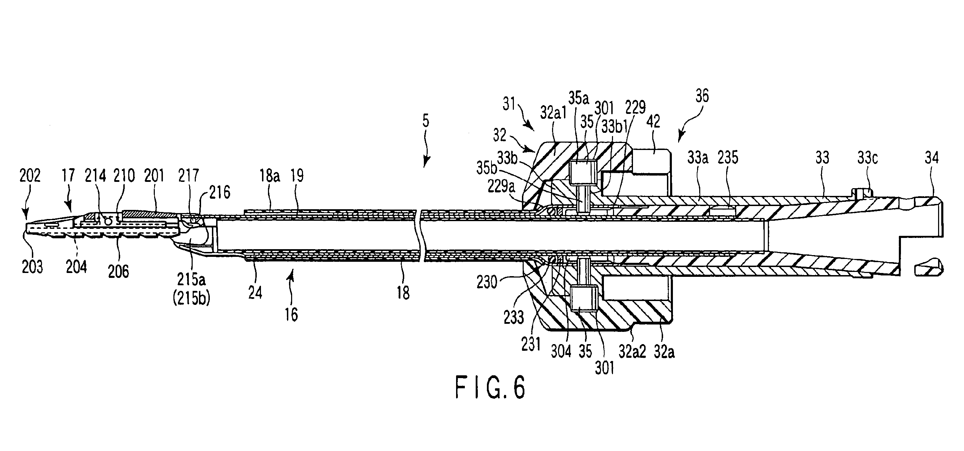

FIG. 6 shows a longitudinal cross-sectional view of the sheath unit 5. The sheath unit 5 includes a sheath main body 16 formed of a cylindrical body, and a jaw 17 arranged at a distal end of the sheath main body 16. The sheath main body 16 includes an outer sheath 18 made of metal which is an external cylinder, and a drive pipe (drive member) 19 made of metal which is an internal cylinder (inner sheath). The drive pipe 19 is inserted into the outer sheath 18 so as to be movable in the axial direction.

An outer circumferential surface of the outer sheath 18 is covered with an outer skin 18a formed of an insulating material such as a resin and the like. An insulating tube 24 formed of an insulating material is arranged on the inner circumferential surface side of the drive pipe 19. A proximal end part of the insulating tube 24 is extended to the proximal end part side of the sheath main body 16. Further, the drive pipe 19 and the probe unit 3 are electrically insulated from each other by the insulating tube 24.

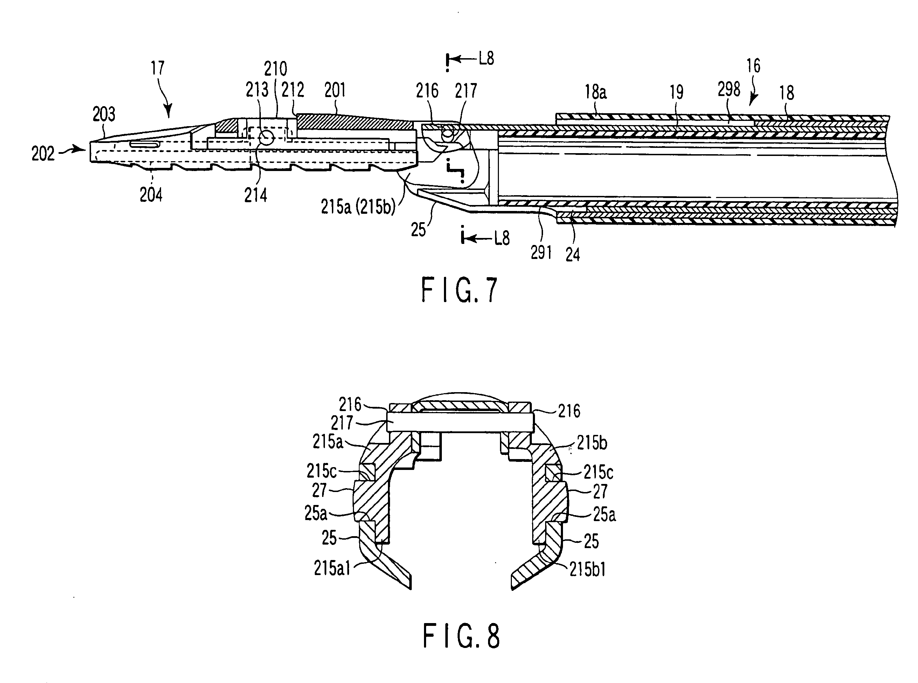

As shown in FIG. 7, a pair of right and left projection pieces 25 (see FIG. 8) are provided to be projected toward the front of the outer sheath 18. As shown in FIG. 8, a circular hole 25a is formed in each of the projection pieces 25. The proximal end part of the jaw 17 is pivotally attached to the circular hole 25a of each of the projection pieces 25 through a boss section 27 (describes later).

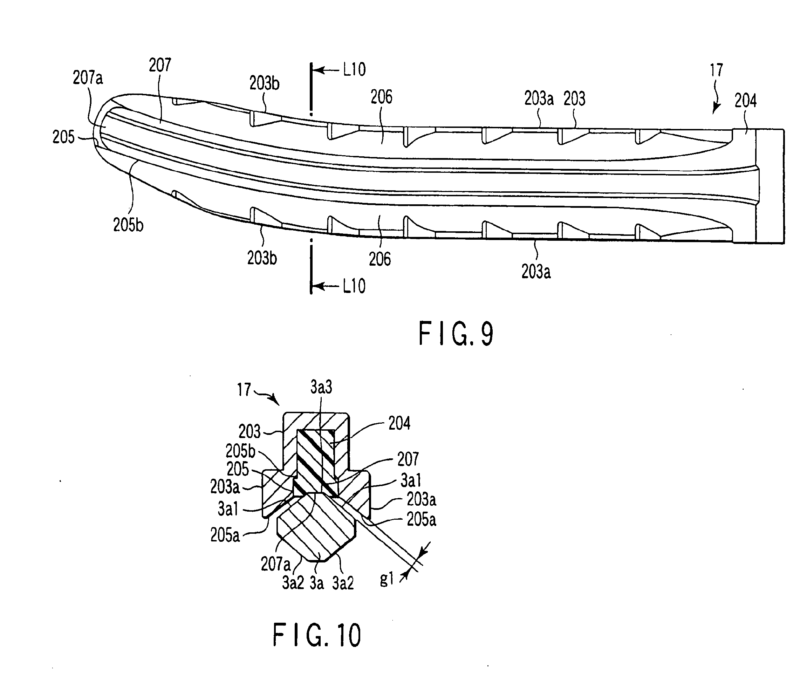

FIG. 9 shows a surface of the jaw 17 at which the jaw is opposed to a probe distal end section 3a. As shown in FIG. 9, the jaw 17 is formed into a curved shape of a substantially J-shape corresponding to the curved shape of the probe distal end section 3a in accordance with the curved shape of the probe distal end section 3a of the probe unit 3. Further, when the probe unit 3 and the sheath unit 5 are coupled to each other, the jaw 17 is arranged at a position at which the jaw 17 is opposed to the probe distal end section 3a of the probe unit 3.

The jaw 17 includes a jaw main body 201 made of metal which is a conductive member, and a grasping member 202 to be attached to the jaw main body 201. The grasping member 202 is constituted of an electrode member 203 for high-frequency treatment, and a pad member 204 (see FIG. 10) for ultrasonic treatment. The electrode member 203 constitutes a second electrode section which is the other of the bipolar electrodes. The pad member 204 is formed of an insulator which is a resin material such as polytetrafluoroethylene and the like.

As shown in FIGS. 9 and 10, a groove section 205 is formed on the undersurface of the electrode member 203 in accordance with the curved shape of the probe distal end section 3a. The pad member 204 is fitted into the groove section 205 in an inserted state.

Inclined surfaces 205a on which the closer a position is to the lower opening surface side, the larger the groove width corresponding to the position becomes are formed on wall surfaces on both sides of the groove section 205 as shown in FIG. 10. Further, as shown in FIG. 9, tooth sections 203b for slip prevention are formed on the lower opening surface side on the wall surfaces 203a on both sides of the groove section 205. These tooth sections 203b constitute a slip prevention section for preventing stuff held between the probe distal end section 3a and the jaw 17 when the jaw 17 and the probe distal end section 3a are engaged with each other from slipping between them. A thickness T of the electrode member 203 is appropriately set in consideration of the rigidity and coagulability.

Further, a notch section 205b is formed at the bottom part of the inclined surfaces 205a of the groove section 205 in the electrode member 203. The notch section 205b is formed in accordance with the curved shape of the probe distal end section 3a. A pressing section 207 of the pad member 204 is arranged in the notch section 205b. As shown in FIG. 10, the pressing section 207 of the pad member 204 is a probe contact member with which the probe distal end section 3a is brought into contact.

An alignment groove 207a is provided at the center of the pressing section 207 of the pad member 204. As shown in FIG. 9, the alignment groove 207a is formed over the entire length of the pad member 204 from the front end part of the pressing section 207 to the rear end part thereof. The probe distal end section 3a is fitted into the alignment groove 207a in an engaged state. Further, in a state where the probe distal end section 3a is fitted into the alignment groove 207a of the pressing section 207 so as to be engaged with the groove 207a, the alignment is performed in a state where the probe distal end section 3a is prevented from being shifted from the electrode member 203 in the lateral direction in FIG. 10. As a result of this, the inclined surfaces 205a of the electrode member 203 and the probe distal end section 3a are prevented from being brought into contact with each other by securing a clearance of a certain distance g1 between the probe distal end section 3a and each of the inclined surfaces 205a of the electrode member 203.

The probe distal end section 3a is formed into a cross-sectional shape shown in FIG. 10. That is, on the top surface side of the probe distal end section 3a, right and left inclined surfaces 3a1 are formed in parallel with the right and left inclined surfaces 205a of the electrode member 203. On the undersurface side of the probe distal end section 3a, right and left inclined surfaces 3a2 are formed in directions opposite to those of the right and left inclined surfaces 3a1. Further, on the top surface side of the probe distal end section 3a, a flat surface section 3a3 parallel with the alignment groove 207a of the pressing section 207 of the pad member 204 is formed between the right and left inclined surfaces 3a1.

Further, the electrode member 203 and the pad member 204 are assembled into an integral body, whereby the grasping member 202 is formed. A projection section 210 for attachment is protrusively provided on the grasping member 202 on the opposite side of the engagement surface 206 at which the jaw 17 is engaged with the probe distal end section 3a. This projection section 210 is screwed onto the jaw main body 201 by means of a fixing screw 214. As a result of this, the grasping member 202 is attached to the jaw main body 201. Here, the electrode member 203 of the grasping member 202 and the jaw main body 201 are electrically connected to each other by means of the fixing screw 214.

The proximal end part of the jaw main body 201 is provided with arm sections 215a and 215b which are formed into two branches. Each of the arm sections 215a and 215b is provided with an extension section 215a1 or 215b1 extending obliquely below from the center line position of the jaw main body 201. As shown in FIG. 8, the boss section 27 is formed on the outer surface of each of the extension sections 215a1 and 215b1 in a state where the boss section is outwardly protruded from the outer surface. Further, the boss section 27 of each of the extension sections 215a1 and 215b1 is engaged with the circular hole 25a formed in each of the right and left projection pieces 25 of the distal end part of the outer sheath 18 in a state where the boss section 27 is inserted into the circular hole 25a. As a result of this, the jaw main body 201 is pivotally attached to the right and left projection pieces 25 of the distal end part of the outer sheath 18 through the boss sections 27.

Further, at a joint part of each of the two arm sections 215a and 215b (upper end part in FIG. 7), a hole 216 into which a connecting pin is to be inserted is formed. A connecting pin 217 for coupling the jaw main body 201 and the drive pipe 19 to each other is fitted in this hole 216. Further, the jaw main body 201 and the drive pipe 19 are electrically connected to each other through the connecting pin 217.

As a result of this, by causing the drive pipe 19 to advance or retreat in the axial direction, the drive force of the drive pipe 19 is transmitted to the jaw 17 through the connecting pin 217. Consequently, the jaw 17 is pivotally driven around the fulcrum pin. At this time, when the drive pipe 19 is backwardly pulled, the jaw 17 is pivotally driven around the fulcrum pin in a direction in which the jaw 17 is separated from the probe distal end section 3a (toward the opened position). Conversely, when the drive pipe 19 is forwardly pushed, the jaw 17 is pivotally driven around the fulcrum pin in a direction in which the jaw 17 is brought closer to the probe distal end section 3a (toward the closed position). The jaw 17 is pivotally driven to the closed position, whereby the living tissue is grasped between the jaw 17 and the probe distal end section 3a of the probe unit 3.

The treatment section 1A of the hand piece 1 is constituted of the jaw 17 and the probe distal end section 3a of the probe unit 3. In the treatment section 1A, a plurality of, for example, in this embodiment, two treatment functions (a first treatment function and a second treatment function) can be selected. For example, the first treatment function is set at a function of simultaneously outputting an ultrasonic treatment output and a high-frequency treatment output. The second treatment function is set at a function of outputting only the high-frequency treatment output alone.

Incidentally, the first treatment function and the second treatment function of the treatment section 1A are not limited to the above configurations. For example, a configuration may be employed in which the first treatment function is set at a function of outputting the ultrasonic treatment output in a maximum output state, and the second treatment function is set at a function of outputting the ultrasonic treatment output in an arbitrarily preset output state which is lower than the maximum output state.

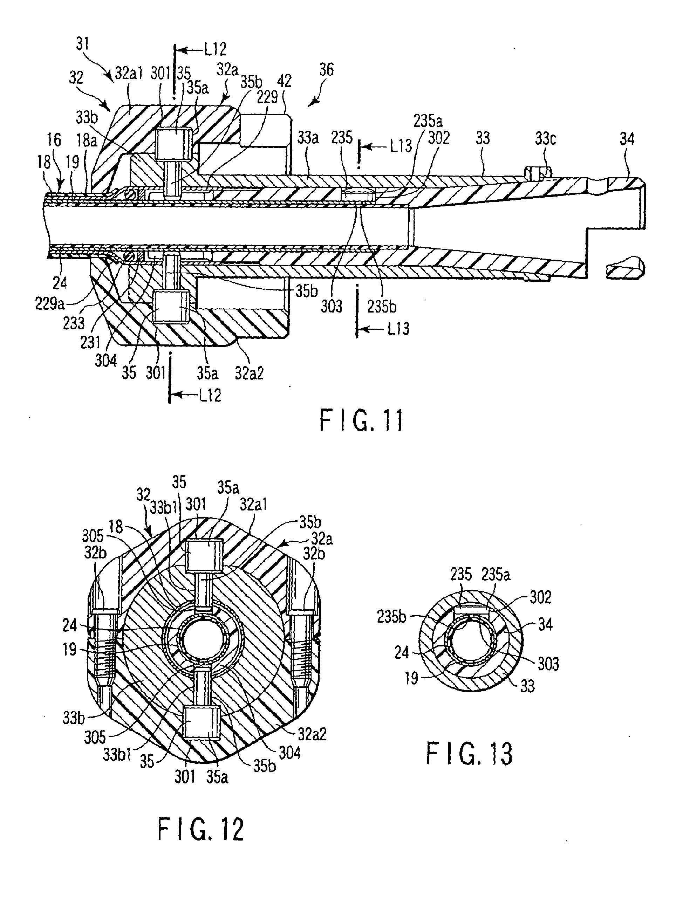

FIG. 11 shows the proximal end part of the sheath main body 16. The proximal end part of the outer sheath 18 is provided with a flare section 229 having an inner diameter larger than that of the other part of the outer sheath 18. The proximal end part of the drive pipe 19 is extended to a position closer to the rear end side than the flare section 229 of the outer sheath 18.

Further, the proximal end part of the sheath main body 16 is provided with an attachment/detachment mechanism section 31 for attaching/detaching the sheath main body 16 to/from the handle unit 4. The attachment/detachment mechanism section 31 is provided with a cylindrical knob member 32 having a large diameter, a guide cylindrical body (first tubular member) 33 constituted of a metallic cylindrical body, and a cylindrical connection tubular body (second tubular member) 34 formed of a resin material.

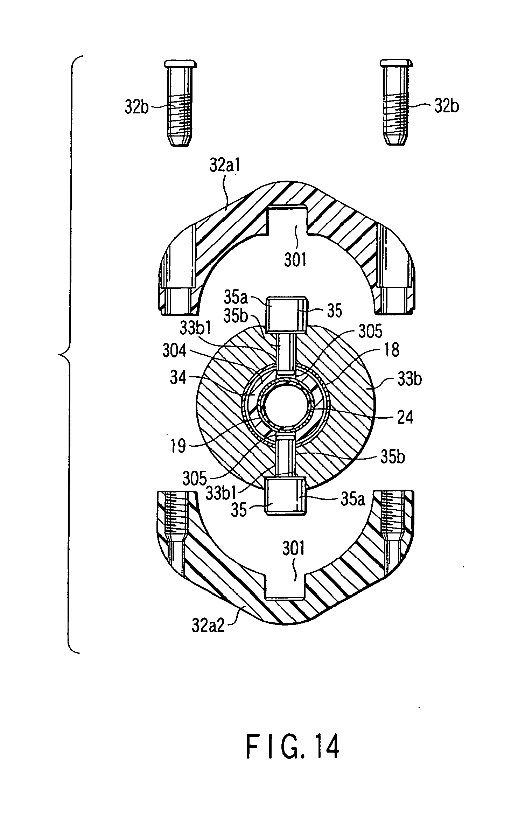

As shown in FIG. 12, the knob member 32 includes a knob main body 32a having a ring-like shape. As shown in FIG. 14, the knob main body 32a includes two C-shaped members 32a1 and 32a2 having a substantially C-shape. These two C-shaped members 32a1 and 32a2 are formed of a resin material, and constitute the knob main body 32a having a ring-like shape in a state where the two C-shaped members 32a1 and 32a2 are coupled to each other at their both ends.

An engagement pit 301 is formed in an inner circumferential surface of each of the two C-shaped members 32a1 and 32a2. A head section 35a of a pin 35 for restraining an internal component from moving is engaged with the engagement pit 301. As a result of this, the position of the pin 35 can be restrained.

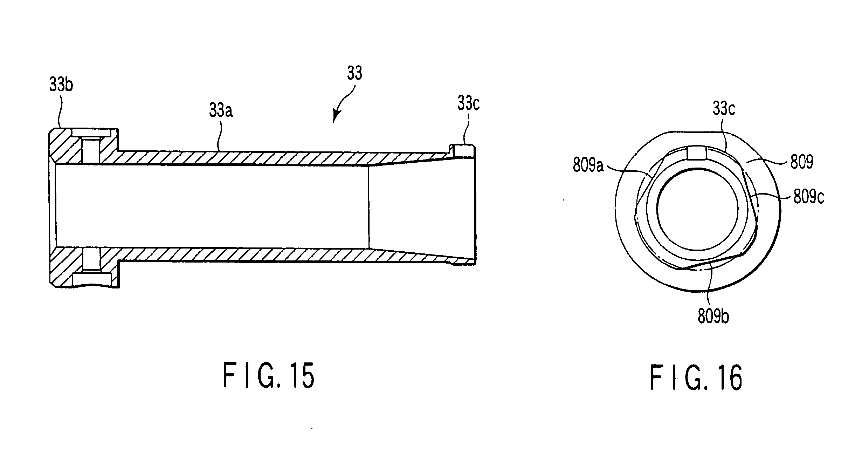

The guide cylindrical body 33 includes a tubular body 33a which is externally fitted on the flare section 229 of the proximal end part of the outer sheath 18, and is backwardly extended. As shown in FIG. 15, the distal end part of the tubular body 33a is provided with a large-diameter section 33b having a larger outer diameter than the other part. The knob member 32 is externally fitted on the large-diameter section 33b. On the outer circumferential surface at the rear end part of the guide cylindrical body 33, an outwardly protruding connection flange section 33c is formed.

In the large-diameter section 33b of the guide cylindrical body 33, two pin-insertion holes 33b1 extending in the radial direction are formed. An axis section 35b of each of the pins 35 is inserted into each of the pin-insertion holes 33b1.

In the flare section 229 of the outer sheath 18, two pin-insertion holes are similarly formed at positions corresponding to the two pin-insertion holes 33b1 of the tubular body 33a. The axis sections 35b of the pins 35 are inwardly protruded through the two pin-insertion holes 33b1 of the tubular body 33a and the two pin-insertion holes of the outer sheath 18. As a result of this, the knob member 32, the guide cylindrical body 33, and the flare section 229 of the outer sheath 18 are assembled into an integral body in a state where the outer sheath 18 is restrained by the pins 35 from moving in the axial direction, and from rotating around the axis.

The connection tubular body 34 is internally fitted into the guide cylindrical body 33 so as to be slidable in the axial direction of the outer sheath 18. A proximal end part of the drive pipe 19 is internally fitted into the inner circumferential surface of the distal end part of the connection tubular body 34.

As shown in FIG. 11, a rotation restraining pin 235 is fixed to the proximal end part of the drive pipe 19. As shown in FIG. 13, the rotation restraining pin 235 includes a large-diameter head section 235a and a small-diameter axis section 235b. In the connection tubular body 34, an engagement hole section 302 to be engaged with the head section 235a of the rotation restraining pin 235 is formed. In the proximal end part of the drive pipe 19, a pin engagement hole 303 to be engaged with the axis section 235b of the rotation restraining pin 235 is formed. Further, the drive pipe 19 and the connection tubular body 34 are coupled to each other by means of the rotation restraining pin 235. At this time, the drive pipe 19 and the connection tubular body 34 are assembled into an integral body in a state where the drive pipe 19 is restrained by the rotation restraining pin 235 from moving in the axial direction, and from rotating around the axis.

The distal end part of the connection tubular body 34 is inserted into the inside of the flare section 229 of the outer sheath 18, and is extended to a position near a step section 229a between the outer sheath 18 and the flare section 229.

Between the flare section 229 and the drive pipe 19, a sealing means 230 for sealing a part between the outer sheath 18 and the drive pipe 19 is provided. The sealing means 230 includes a backup ring 231 and an O-ring 233. The O-ring 233 is provided between the step section 229a of the flare section 229 and the backup ring 231 so as to be movable in the axial direction of the outer sheath 18. Further, the position of the backup ring 231 of the O-ring 233 is restrained on the distal end part of the connection tubular body 34. Further, by utilizing the shape of the step section 229a of the flare section 229, it is possible to cause the step section 229a to double as a backup ring on the front side of the O-ring 233. As a result of this, it is possible to make the number of backup rings 231 of the O-ring 233 only one.

The distal end part of the connection tubular body 34 includes two slits 305 extended in the axial direction of the drive pipe 19. An inner end part of the axis section 35b of each of the pins 35 is inserted in and engaged with each of these slits 305. As a result of this, it is possible to restrain the three parts including the guide cylindrical body 33, the outer sheath 18, and the connection tubular body 34 from moving in the rotational direction with respect to the knob member 32 by the pins 35.

At a rear end part of the knob member 32, an attachment/detachment section 36 for attaching/detaching the knob member 32 to/from the handle unit 4 is arranged. The attachment/detachment section 36 of the knob member 32 is provided with a guide groove (not shown) having an inclined surface-like shape, and an engagement concave section 42. The guide groove is extended in the circumferential direction on the outer circumferential surface of the proximal end part of the knob member 32. Further, the guide groove includes a taper-like inclined surface on which the outer diameter becomes smaller as the diameter becomes closer to the rear end part side of the knob member 32.

The engagement concave section 42 is formed at one end part of the guide groove. The engagement concave section 42 is constituted of a depression section having a diameter smaller than the width of the inclined surface of the guide groove. An engagement lever 43 to be described later on the handle unit 4 side is disengageably engaged with the engagement concave section 42.

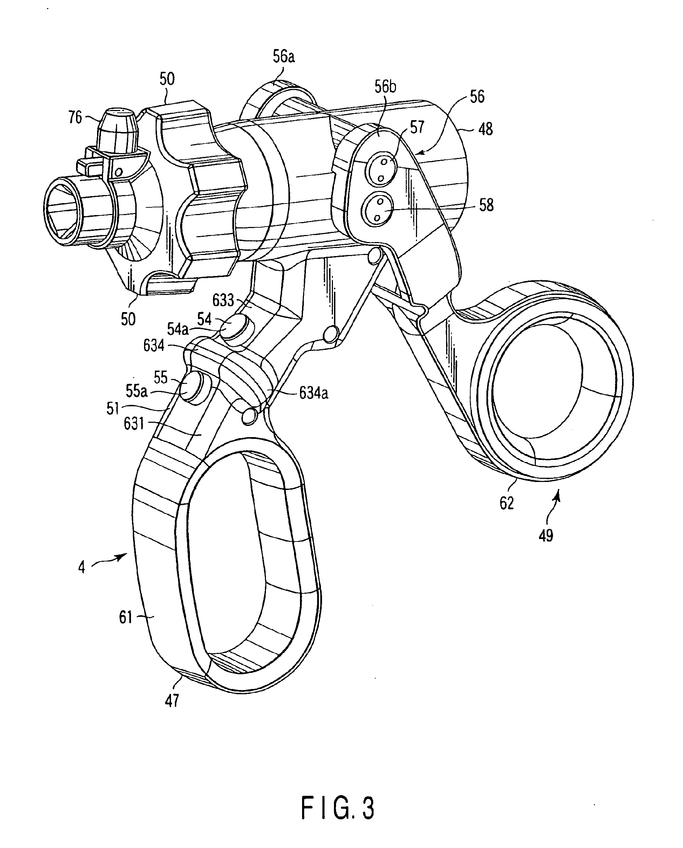

As shown in FIG. 3, the handle unit 4 is mainly includes a fixed handle 47, a retaining cylinder 48, a movable handle 49, and a rotary operation knob 50. The fixed handle 47 includes a plural finger insertion ring section 61 in which, for example, a plurality of fingers of a user other than a thumb is inserted.

As shown in FIG. 4, the fixed handle 47 of this embodiment includes a handle main body 631 in which the retaining cylinder 48 side and the plural finger insertion ring section 61 are formed integral with each other. The handle main body 631 of the fixed handle 47 is provided with a switch holding section 51 between the plural finger insertion ring section 61 and the retaining cylinder 48.

As shown in FIG. 3, the switch holding section 51 includes a switch attachment surface 633 to which a plurality of, for example, in this embodiment, two hand switches (a first switch 54 and a second switch 55) are attached. Each of these first switch 54 and second switch 55 is a switch for selecting a treatment function of the treatment section 1A of the hand piece 1.

In the switch holding section 51, the first switch 54 and the second switch 55 are arranged in the vertical direction. Further, a protuberance section 634 which serves a partition wall doubling as a finger receiver is formed between the first switch 54 and the second switch 55.

The first switch 54 is arranged on the upper side of the protuberance section 634. The first switch 54 is set as a switch for selecting the first treatment function having the highest frequency in use among the plural treatment functions described previously.

The second switch 55 is arranged on the downside of the protuberance section 634. The second switch 55 is set as a switch for selecting the second treatment function which is another of the plural treatment functions described previously. For example, the first switch 54 is set as a switch button for incision, and the second switch 55 is set as a switch button for coagulation.

The protuberance section 634 is set in such a manner that a height of the protrusion thereof from the switch attachment surface 633 is larger than those of the first switch 54 and the second switch 55. The protuberance section 634 includes an extension section 634a continuously extended from the switch attachment surface 633 of the fixed handle 47 toward both side surfaces.

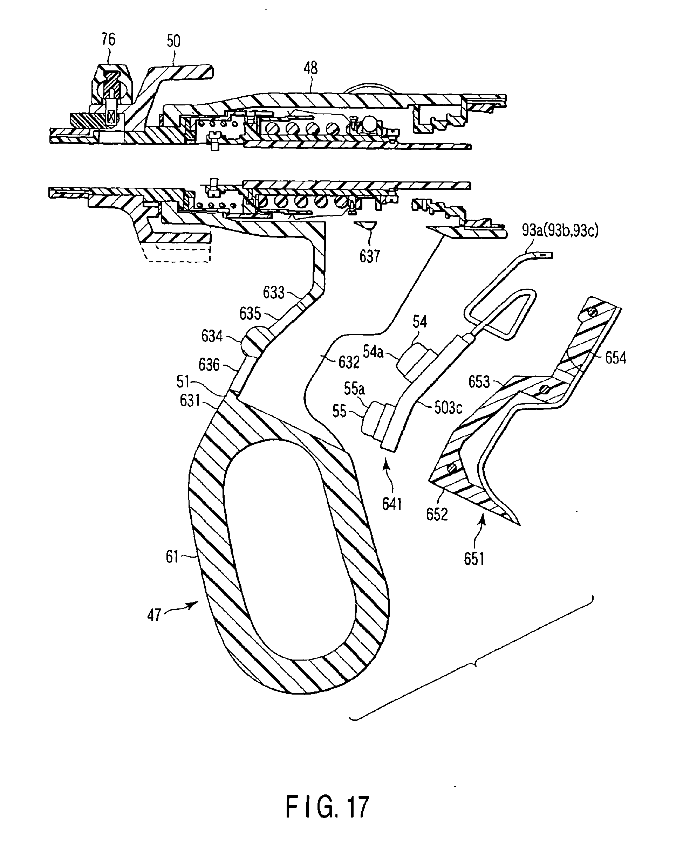

As shown in FIG. 17, the handle main body 631 of the fixed handle 47 includes a concave section 632 on the part of the switch holding section 51 at which the rear part side of the handle main body 631 is opened. The switch attachment surface 633 is formed on the front wall part of the concave section 632.

On or in the switch attachment surface 633, the protuberance section 634, a first switch button insertion hole 635, and a second switch button insertion hole 636 are formed. The first switch button insertion hole 635 is arranged on the upper side of the protuberance section 634. The second switch button insertion hole 636 is arranged on the downside of the protuberance section 634.

As shown in FIGS. 4A, and 4B, a switch unit 641 and a switch pressing member 651 are fixed to the concave section 632 of the handle main body 631 in a state where the switch unit 641 and the pressing member 651 are inserted therein. The switch unit 641 is formed by integrating the two switches (the first switch 54 and second switch 55) into one unit as shown in FIG. 17.

The switch unit 641 includes a push button 54a for the first switch 54, a push button 55a for the second switch 55, a flexible wiring circuit board 503a for the two switches (the first switch 54 and second switch 55), and a flexible base member 503c in which the wiring circuit board 503a is embedded in two insulating rubber plates (elastic bodies) 503b.

Wiring 93a for the first treatment function one end of which is connected to the first switch 54, wiring 93b for the second treatment function one end of which is connected to the second switch 55, and wiring 93c for the ground one end of which is connected to a common terminal for the ground are connected to the wiring circuit board 503a. These three pieces of the wiring 93a to 93c are contained inside the concave section 632 of the handle main body 631 in a state where the pieces of the wiring are rolled up.

In the switch unit 641, the push button 54a for the first switch 54 is inserted into the first switch button insertion hole 635, and the push button 55a for the second switch 55 is inserted into the second switch button insertion hole 636. In this state, the base member 503c of the switch unit 641 is attached to the concave section 632 of the handle main body 631 in a state where the base member 503c is pressed against the switch attachment surface 633 side from the rear end side by the switch pressing member 651.

As shown in FIG. 17, the switch pressing member 651 includes a guide surface 652, a convex section 653 for pressing the switch unit, and a wiring holding section 654. The guide surface 652 is joined to the concave section 632 of the handle main body 631 along the downside wall surface thereof in FIG. 17.

The convex section 653 for pressing the switch unit presses the base member 503c of the switch unit 641 against the switch attachment surface 633 side. At this time, the base member 503c of the switch unit 641 is tightly pressed against the switch attachment surface 633 side in a state where the base member 503c is bent by the convex section 653 for pressing the switch unit. As a result of this, the base member 503c itself of the switch unit 641 fulfills a function of a gasket, and hence the sealing member or the like around the switch unit 641 can be reduced.

The wiring holding section 654 holds the wiring pieces 93a, 93b, and 93c of the switch unit 641 in the concave section 632 of the handle main body 631.

Further, the handle main body 631 is provided with a boss section 637 between the concave section 632 and the internal space of the retaining cylinder 48 in a protruding manner. This boss section 637 prevents the wiring pieces 93a, 93b, and 93c of the switch unit 641 from entering the internal space side of the retaining cylinder 48 to interfere with the internal operation members in the retaining cylinder 48.

The movable handle 49 includes an arm section 56 having a substantially U-shape at an upper part thereof. At a lower part of the movable handle 49, a finger insertion ring section 62 in which, for example, a thumb of the user is inserted is provided. A ring-shaped finger contact member 62a with a heat-resistant rubber lining is attached to the finger insertion ring section 62.

The U-shaped arm section 56 includes two arms 56a and 56b. The movable handle 49 is attached to the retaining cylinder 48 in a state where the retaining cylinder 48 is inserted between the two arms 56a and 56b.

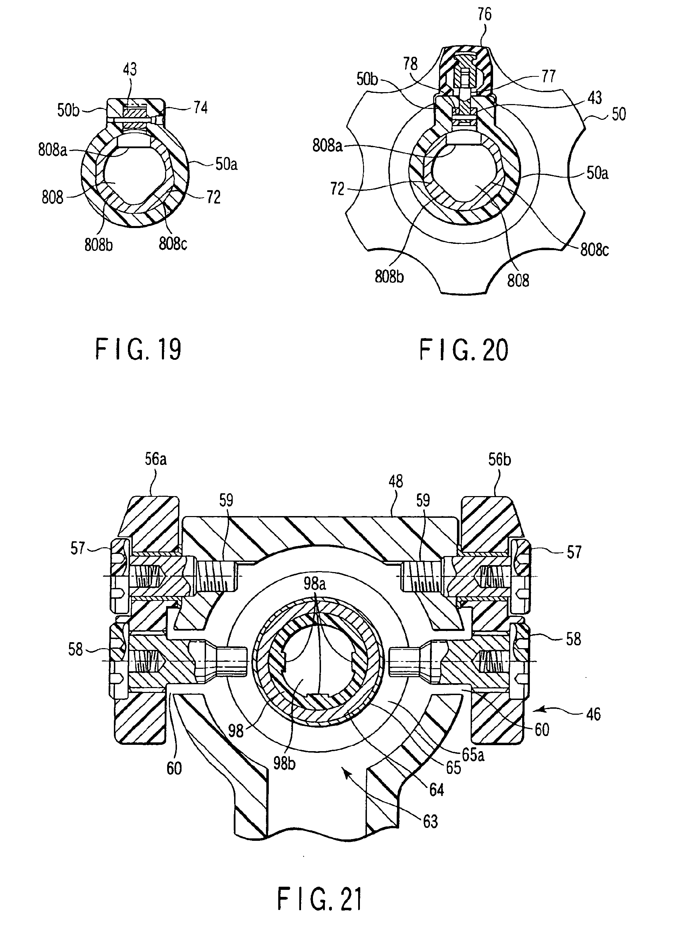

Each of the arms 56a and 56b includes a fulcrum pin 57 and an action pin 58. As shown in FIG. 21, on both sides of the retaining cylinder 48, a pin receiving hole section 59 and a window section 60 are formed. The fulcrum pin 57 of each of the arms 56a and 56b is inserted in the pin receiving hole section 59 of the retaining cylinder 48. As a result of this, an upper part of the movable handle 49 is pivotally supported by the retaining cylinder 48 through the fulcrum pins 57. Further, the movable handle 49 is turned around the fulcrum pins 57, and the movable handle 49 is operated to be opened or closed with respect to the fixed handle 47.

Each of the action pins 58 of the movable handle 49 is extended through the window section 60 of the retaining cylinder 48 to the inside of the retaining cylinder 48. A operation force transmission mechanism 63 for transmitting the operation force of the movable handle 49 to the drive pipe 19 of the jaw 17 is provided inside the retaining cylinder 48.

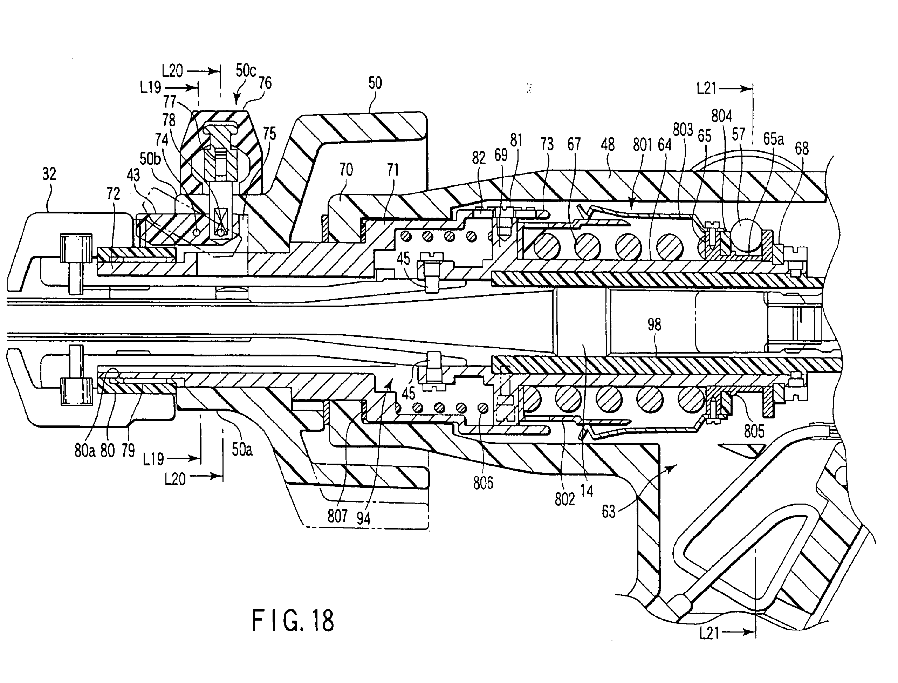

As shown in FIG. 18, the operation force transmission mechanism 63 mainly includes a slider receiving member 64 made of metal and having a cylindrical shape, and a slider member 65. The slider receiving member 64 is arranged concentric with the center line of the retaining cylinder 48, and is extended in the same direction as the insertion direction of the probe unit 3.

A stopper 68, and a spring receiver 69 are arranged on the outer circumferential surface of the slider receiving member 64. The stopper 68 is fixed to the outer circumferential surface of the proximal end part of the slider receiving member 64. The spring receiver 69 is provided on the outer circumferential surface on the distal end part side of the slider receiving member 64 so as to be protruded. Between the stopper 68 and the spring receiver 69, the slider member 65 and a coil spring 67 are arranged. The stopper 68 restrains the movement position of the slider member 65 on the rear end side. The spring receiver 69 is in contact with the front end part of the coil spring 67. The coil spring 67 is arranged between the spring receiver 69 and the slider member 65 with a certain amount of equipped force.

A ring-shaped engagement groove 65a is formed on the outer circumferential surface of the slider member 65 in the circumferential direction. The action pin 58 of the movable handle 49 is engaged with the engagement groove 65a in a state where the action pin 58 is inserted in the engagement groove 65a as shown in FIG. 21. Further, when the movable handle 49 is gripped, and the movable handle 49 is closed with respect to the fixed handle 47, the action pin 58 is rotated around the fulcrum pin 57 concomitantly with the turning operation of the movable handle 49 at this time. The slider member 65 is moved forward in the axial direction (the same direction as the insertion direction of the probe unit 3) concomitantly with the operation of the action pin 58. At this time, the slider receiving member 64 coupled to the slider member 65 through the coil spring 67 is also advanced or retreated together with the slider member 65.

A pair of engagement pins 45 used when the sheath unit 5 and the handle unit 4 side are attached/detached to/from are fixed to the distal end part of the slider receiving member 64. As a result of this, the operation force of the movable handle 49 is transmitted to the connection tubular body 34 of the sheath unit 5 through the pair of engagement pins 45, and the drive pipe 19 of the jaw 17 is moved in the forward direction. Therefore, the jaw main body 201 of the jaw 17 is turned around the fulcrum pin.

Further, when the living tissue is grasped between the grasping member 202 of the jaw 17 and the probe distal end section 3a of the probe unit 3 by this operation, the grasping member 202 is turned by a certain amount of angle around the fixing screw 214 as a fulcrum following the bending of the probe distal end section 3a, whereby the force is uniformly applied to the grasping member 202 over the entire length of the grasping member 202. In this state, by outputting the ultrasonic waves, coagulation or incision of the living tissue such as a blood vessel is enabled.

A ring-shaped bearing section 70 is formed at the front end part of the retaining cylinder 48. A cylindrical rotation transmission member 71 made of metal is coupled to the bearing section 70 so as to be rotatable in a direction around the axis. On the rotation transmission member 71, a protrusion section 72 forwardly protruded toward the front of the bearing section 70, and a large-diameter section 73 extended from the bearing section 70 toward the inside of the retaining cylinder 48 are formed.

The rotary operation knob 50 is fixed to the protrusion section 72 in a state where the rotary operation knob 50 is externally fitted on the protrusion section 72. A fixed ring section 50a having a small diameter is formed at the front end part of the rotary operation knob 50. An outward protrusion section 50b outwardly protruding in the radial direction is formed on a part of the outer circumferential surface of the fixed ring section 50a as shown in FIGS. 19 and 20. The outward protrusion section 50b is provided with an attachment/detachment operation section 50c used for attaching/detaching the handle unit 4 to/from the sheath unit 5.

The attachment/detachment operation section 50c is provided with the engagement lever 43 to be disengageably engaged with the engagement concave section 42 of the knob member 32 of the sheath unit 5. The middle part of the engagement lever 43 is pivotally coupled to the outward protrusion section 50b of the rotary operation knob 50 through a pin 74 as shown in FIG. 19. A proximal end part of the engagement lever 43 is extended to the inside of a lever receiving concave section 75 formed in the front of the rotary operation knob 50.

The attachment/detachment operation section 50c of the rotary operation knob 50 is provided with an operation button 76 used to operate the engagement lever 43 in the engagement releasing direction. As shown in FIG. 20, the operation button 76 is provided with a downwardly set operation pin 77 in a protruding manner. The operation pin 77 is extended through a hole in the wall of the outward protrusion section 50b of the rotary operation knob 50 to the inside of the lever receiving concave section 75. A proximal end part of the engagement lever 43 is pivotally coupled to the lower end part of the operation pin 77 through a pin 78.

A falling-off prevention ring 80 to prevent the rotary operation knob 50 from falling off is provided at the distal end part of the protrusion section 72 of the rotation transmission member 71. Here, a male thread section 79 is formed at the distal end part of the protrusion section 72. A female thread section 80a to be screw-engaged with the male thread section 79 is formed on the inner circumferential surface of the falling-off prevention ring 80. Further, the female thread section 80a of the falling-off prevention ring 80 is joined to the male thread section 79 of the protrusion section 72 in a screwing manner, whereby the rotary operation knob 50 is fixed to the rotation transmission member 71.

A positioning pin 81 made of metal is attached to the spring receiver 69 of the slider receiving member 64 so as to be outwardly protruded in the radial direction. An engagement hole section 82 having a shape of an elongate hole in which the one pin 81 of the slider receiving member 64 is inserted is formed in the large-diameter section 73 of the rotation transmission member 71. The engagement hole section 82 is extended in the same direction as the insertion direction of the probe unit 3. As a result of this, when the movable handle 49 is operated, the advancing/retreating operation of the slider receiving member 64 is prevented from being transmitted to the rotation transmission member 71 by moving the pin 81 along the engagement hole section 82.

On the other hand, when the rotary operation knob 50 is operated to be rotated, the rotation operation of the rotation transmission member 71 which rotates together with the rotary operation knob 50 is transmitted to the slider receiving member 64 side through the pin 81. As a result of this, when the rotary operation knob 50 is operated to be rotated, the rotation transmission member 71, pin 81, slider receiving member 64, slider member 65, and coil spring 67 which are in the retaining cylinder 48, and are assembled into a unit are rotationally driven as one body in the direction around the axis together with the rotary operation knob 50.

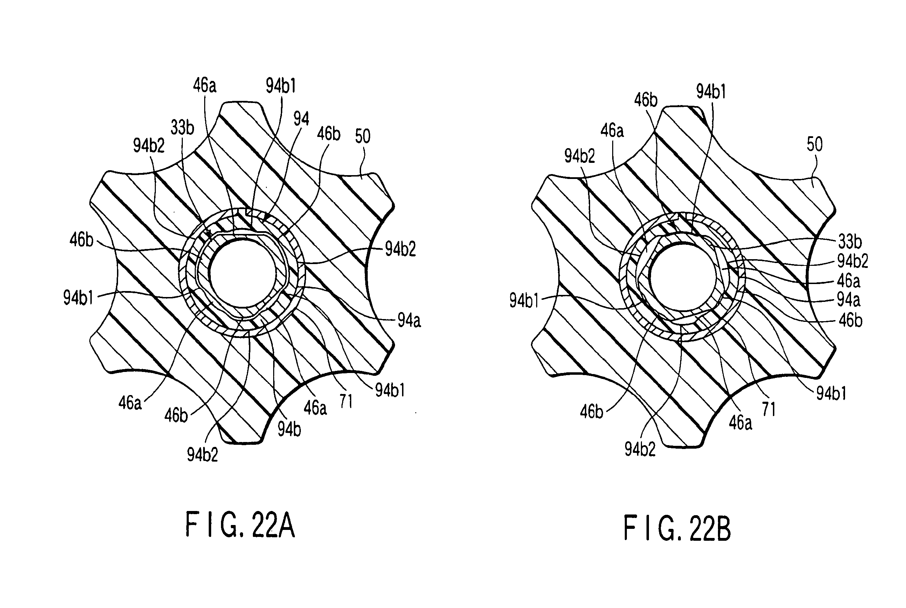

An engagement means 94 which is disengageably engaged with the connection flange section 33c of the sheath unit 5 is provided on the inner circumferential surface of the rotation transmission member 71. FIGS. 22A and 22B show the engagement means 94. This engagement means 94 includes an insertion hole section 94a in which the connection flange section 33c is inserted when the sheath unit 5 and the handle unit 4 are coupled to each other, and a conductive rubber ring (energizing means) 94b arranged in the insertion hole section 94a.

The shape of the inner circumferential surface of the conductive rubber ring 94b is substantially the same as that of an engagement section 46 of the connection flange section 33c. That is, on the inner circumferential surface of the conductive rubber ring 94b, three flat sections 99b1 formed by flattening a plurality of, for example, in this embodiment, three parts of the circular inner circumferential surface, and three corner sections 94b2 arranged at three joint sections between the three flat sections 99b1, and having a larger diameter than the flat sections 94b1 are formed. As a result of this, the conductive rubber ring 94b is formed into a substantially triangular cross-sectional shape. Thus, when the shape of the inner circumferential surface of the conductive rubber ring 94b and the shape of the engagement section 46 of the connection flange section 33c correspond to each other as shown in FIG. 22A, i.e., in a state where the three corner sections 46b of the connection flange section 33c and the three corner sections 94b2 of the conductive rubber ring 94b coincide with each other, the conductive rubber ring 94b is held at a non-compression position in a natural state. On the other hand, when between the handle unit 4 and the sheath unit 5 is relatively rotated in the direction around the center axis of the sheath unit 5, the position of the conductive rubber ring 94b is switched to a compression contact position at which the conductive rubber ring 94b is brought into compression contact with the three corner sections 46b of the connection flange section 33c as shown in FIG. 22B. At this time, the three corner sections 46b of the connection flange section 33c are brought into contact with the three flat sections 94b1 of the conductive rubber ring 94b, whereby the conductive rubber ring 94b is compressed.

In this embodiment, when the sheath unit 5 and the handle unit 4 are coupled to each other, at the insertion operation time when the connection flange section 33c of the sheath unit 5 is inserted straight in the inside of the conductive rubber ring 94b, the conductive rubber ring 94b is held at a non-compression position in the natural state as shown in FIG. 22A. At this time, the engagement lever 43 on the handle unit 4 side is held in a state where the lever 43 runs on the inclined surface of the guide groove of the knob member 32 of the sheath unit 5. Thereafter, by rotating the knob member 32 of the sheath unit 5 in the direction around the axis with respect to the handle unit 4, the engagement lever 43 on the handle unit 4 side is engaged with the engagement concave section 42 at one end part of the guide groove in a state where the engagement lever 43 is inserted in the engagement concave section 42. At this time, the position of the conductive rubber ring 94b is switched to the compression contact position at which the conductive rubber ring 94b is brought into compression contact with the three corner sections 46b of the connection flange section 33c as shown in FIG. 22B. As a result of this, the sheath unit side electric pathway 40 (formed between each of the guide cylindrical body 33, fixing screw 39, joint pipe 38, sheath 18, end cover 25, fulcrum pin, and jaw main body 28) and the handle unit side electric pathway 95 (formed between each of the electric contact point member 96, slider receiving member 64, coil spring 806, and rotation transmission member 71) are electrically connected to each other through the conductive rubber ring 94b. At this time, in the connected body of the sheath unit 5 and the handle unit 4, a second high-frequency electric pathway 97 through which a high-frequency current is transmitted is formed.

The handle unit 4 includes a tubular member 98 formed on the inner circumferential surface of the slider receiving member 64 made of an insulating material. The tubular member 98 is fixed to the inner circumferential surface of the slider receiving member 64. As a result of this, when the probe unit 3 and the handle unit 4 are connected to each other, a first high-frequency electric pathway 13 and the second high-frequency electric pathway 97 are insulated from each other by the tubular member 98.

As shown in FIG. 21, on the inner circumferential surface of the tubular member 98, convex sections 98a are formed at positions corresponding to the three engagement concave sections 15 of the flange section 14 of the probe unit 3. As a result of this, an oddly shaped engagement hole section 98b corresponding to the oddly shaped part of the flange section 14 of the probe unit 3 is formed on the inner circumferential surface of the tubular member 98. When the probe unit 3 and the handle unit 4 are connected to each other, the oddly shaped part of the flange section 14 of the probe unit 3 and the oddly shaped engagement hole section 98b of the tubular member 98 are disengageably engaged with each other. As a result of this, the positions of the probe unit 3 and the tubular member 98 of the handle unit 4 in the rotational direction are restrained. Therefore, when the rotary operation knob 50 is rotated to be operated, the assembled unit inside the retaining cylinder 48, together with the connected body of the probe unit 3 and the transducer unit 2 are driven to be rotated as one body.

Incidentally, the engagement section between the flange section 14 of the probe unit 3 and the tubular member 98 is not limited to the configuration described above. For example, the tubular member 98 may be formed into a cross-sectional shape of a D-shape, and the flange section 14 of the probe unit 3 may be formed into a cross-sectional shape of a D-shape corresponding to the above cross-sectional D-shape.

Further, in the hand piece 1 of this embodiment, a notification mechanism 801 for notifying of a state where the slider member 65 of the operation force transmission mechanism 63 is moved by an amount equal to or larger than the set amount when the movable handle 49 of the handle unit 4 is operated is incorporated.

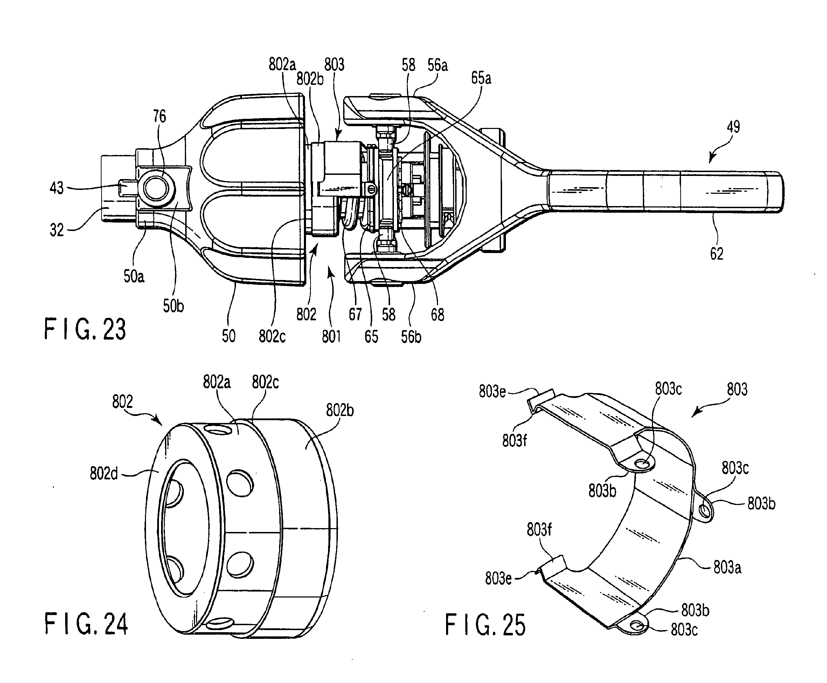

The notification mechanism 801 includes a cylindrical body 802 shown in FIG. 24 and a leaf spring member 803 shown in FIG. 25. The cylindrical body 802 is fixed to the slider receiving member 64 side for guiding the movement of the slider member 65. The leaf spring member 803 is fixed to the slider member 65.

The cylindrical body 802 includes two cylindrical sections 802a and 802b having different diameters. A diameter of the first cylindrical section 802a is formed larger than the diameter of the coil spring 67. The second cylindrical section 802b is formed larger in diameter than the first cylindrical section 802a. Between the first cylindrical section 802a and the second cylindrical section 802b, a step section 802c at which the outer diameter is changed is formed.

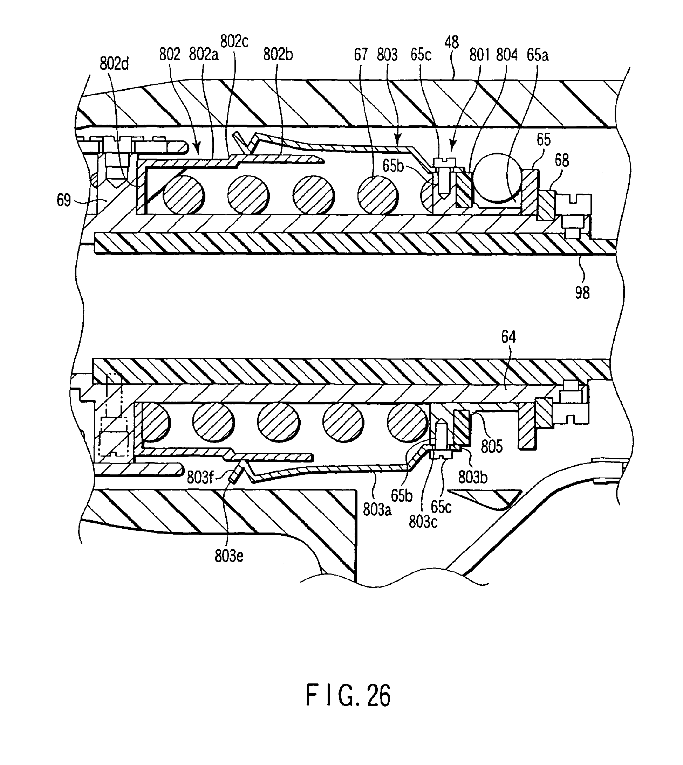

At one end part (end part on the opposite side of the step section 802c) of the first cylindrical section 802a, a joint ring 802d to be joined to the spring receiver 69 of the slider receiving member 64 is provided. As shown in FIG. 26, the cylindrical body 802 is fixed to the slider receiving member 64 side in a state where the joint ring 802d is joined to the spring receiver 69 of the slider receiving member 64.

The leaf spring member 803 includes a leaf spring member main body 803a formed by bending a leaf spring into a substantially semicircular shape. A plurality of, for example, in this embodiment, three bent pieces 803b for fixation are provided at one end part of the leaf spring member main body 803a. A pin insertion hole 803c is formed in each of the bent pieces 803b. Two protrusion pieces 803d that forwardly protrude are provided at the other end part of the leaf spring member main body 803a. A pressure contact section 803e bent inwardly into a substantially V-shape is formed on each of the protrusion pieces 803d.

In the leaf spring member 803, the three bent pieces 803b for fixation are joined to the front end part of the slider member 65. Three screw holes 65b are formed in the front end part of the slider member 65. A screw part of a fixing screw 65c is inserted in the pin insertion hole 803c of each of the bent pieces 803b, and is then screwed into each of the screw holes 65b to be fixed. Here, the pin insertion hole 803c of each of the bent pieces 803b is formed larger in diameter than the screw part of the fixing screw 65c. As a result of this, the leaf spring member 803 is fixed to the front end part of the slider member 65 in a loose-fit state. Further, the pressure contact sections 803e of the leaf spring member main body 803a are set in a state where the sections 803e are in pressure contact with the outer circumferential surface of the cylindrical body 802.

In the notification mechanism 801, when the slider member 65 is operated, the leaf spring member 803 is moved together with the slider member 65, in the same direction as the slider member 65. Further, when the pressure contact sections 803e of the leaf spring member 803 pass the step section 802c between the first cylindrical section 802a and the second cylindrical section 802b, the pressure contact sections 803e fall over the step section 802c. At this time, a knocking sound is generated by the pressure contact sections 803e of the leaf spring member 803 knocking the circumferential wall surface of the first cylindrical section 802a on the lower side of the step section 802c, whereby the state where the slider member 65 of the operation force transmission mechanism 63 has been moved by an amount equal to or larger than a set amount is notified of.

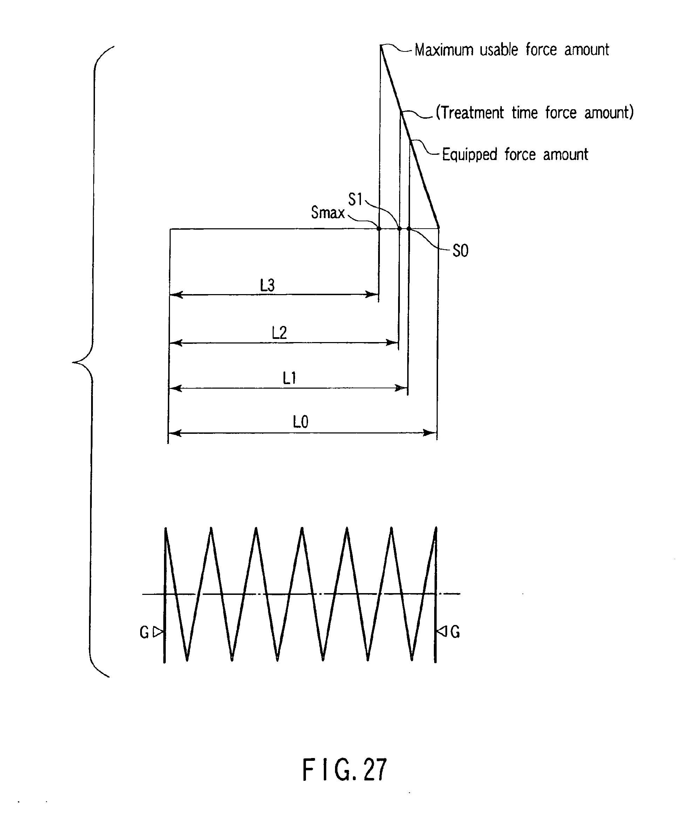

Further, FIG. 27 is an explanatory view for explaining the deformation state of the coil spring 67 at the operation time of the slider member 65. In FIG. 27, a length L0 is a natural length of the coil spring 67, and a length L1 is a set length at the time when the coil spring 67 is set between the spring receiver 69 of the operation force transmission mechanism 63 and the slider member 65 at a certain equipped force amount. In this state, a stroke S of the slider member 65 is S0.

Thereafter, when the movable handle 49 is manipulated in a direction in which the handle 49 is closed, the operation force is transmitted to the slider member 65 according to the operation of the movable handle 49. At this time, the action pin 58 is rotated around the fulcrum pin 57 concomitantly with the turning operation of the movable handle 49. The slider member 65 is moved forward in the axial direction (the same direction as the insertion direction of the probe unit 3) concomitantly with the operation of the action pin 58. At this time, the slider receiving member 64 coupled to the slider member 65 through the coil spring 67 is also advanced or retreated together with the slider member 65.

Further, when the operation force is equal to or larger than a certain amount of the operation force set in advance, the slider receiving member 64 is brought into contact with the stopper, and hence the forward movement is stopped. For this reason, thereafter, only the slider member 65 moves in the forward direction against the spring force of the coil spring 67 concomitantly with the turning operation of the movable handle 49.

Thereafter, at a point of time at which the grasping force for grasping the living tissue between the grasping member 202 of the jaw 17 and the probe distal end section 3a of the probe unit 3 has reached the appropriate set value, the grasping force amount becomes the treatment time force amount. At this time, the stroke S of the slider member 65 is S1, and the length of the coil spring 67 is L2. Incidentally, when the movable handle 49 has reached the maximum turning operation position (maximum usable force amount), the stroke S of the slider member 65 is Smax, and the length of the coil spring 67 is L3.

Further, the notification mechanism 801 of this embodiment is set in such a manner that at a point of time at which the stroke S of the slider member 65 has become S1, and the length of the coil spring 67 has changed to L2, the pressure contact sections 803e of the leaf spring member 803 generate a knocking sound by knocking the circumferential wall surface of the first cylindrical section 802a on the lower side of the step section 802c.

Further, the hand piece 1 of this embodiment is provided with a frictional force reduction section 821 at a contact part at which an inner wall surface of the engagement groove 65a of the slider member 65 and each of the action pins 58 of the movable handle 49 are in contact with each other. This frictional force reduction section 821 includes a ring-shaped contact member 804 formed of a slippery resin member, for example, a PTFE (polytetrafluoroethylene) resin material such as Teflon (registered trade name), and fixed to the inner wall surface of the engagement groove 65a of the slider member 65. This contact member 804 is arranged on the front side of the action pin 58 of the movable handle 49 inserted in the engagement groove 65a. On the bottom part of the engagement groove 65a, a fixing projection 805 for fixing the contact member 804 to the wall surface on the front side of the engagement groove 65a is provided to be projected.

Further, when the pressing force is applied from the action pin 58 to the wall surface on the front side of the engagement groove 65a, the action pin 58 is brought into contact with the contact member 804. As a result of this, the action pin 58 is prevented from being brought into direct contact with the metal surface of the wall surface on the front side of the engagement groove 65a. Accordingly, in a state where the movable handle 49 is closed with respect to the fixed handle 47, and the living tissue is grasped between the grasping member 202 of the jaw 17 and the probe distal end section 3a of the probe unit 3, the rotation force amount of the rotary operation knob 50 can be made small when the rotary operation knob 50 is operated to be rotated.

Further, the hand piece 1 of this embodiment is provided with a coil spring 806 made of metal or the like and having conductivity between the front surface of the slider member 65 and the rear end surface of the protrusion section 72 of the rotation transmission member 71. By virtue of this coil spring 806, a stable high-frequency current pathway can be secured between the front surface of the slider member 65 and the rotation transmission member 71. Furthermore, the spring force of this coil spring 806 can be made to function as the energizing force for automatically opening the closed movable handle 49.

Further, the hand piece 1 of this embodiment is provided with a bearing section 70 of the retaining cylinder 48 of the fixed handle 47, and a ring-shaped washer 807 formed of a slippery resin member such as Teflon (registered trade name) at the contact surface between the bearing section 70 and the rotation transmission member 71. As a result of this, when rotary operation knob 50 is operated to be rotated, the frictional force acting at the contact surface between the bearing section 70 of the retaining cylinder 48 of the fixed handle 47 and the rotation transmission member 71 can be made small. Thus, the rotation force amount of the rotary operation knob 50 can be made small when the rotary operation knob 50 is operated to be rotated.

Furthermore, as shown in FIGS. 19 and 20, in the hand piece 1 of this embodiment, an oddly shaped hole section 808 having a noncircular shape is formed on the inner circumferential surface of the protrusion section 72 of the rotation transmission member 71. This oddly shaped hole section 808 is constituted of a substantially triangular hole section having three flat surfaces 808a, 808b, and 808c on a circular inner circumferential surface.

Further, as shown in FIG. 16, on the connection flange section 33c of the guide cylindrical body 33 of the sheath unit 5, an oddly shaped engagement section 809 having a noncircular shape corresponding to the oddly shaped hole section 808 is formed. This oddly shaped engagement section 809 is constituted of a substantially triangular flange section having three flat surfaces 809a, 809b, and 809c on a circular outer circumferential surface of the connection flange section 33c.

Further, when the sheath unit 5 and the handle unit 4 are coupled to each other, in a correct case of a combination of units of the same type, the oddly shaped hole section 808 of the rotation transmission member 71 of the handle unit 4 and the oddly shaped engagement section 809 of the guide cylindrical body 33 of the sheath unit 5 can be correctly engaged with each other to be normally coupled to each other.

Next, the function of this embodiment will be described below. The hand piece 1 of the surgical operating apparatus of this embodiment can be disassembled so as to be divided into four units of the transducer unit 2, probe unit 3, handle unit 4, and sheath unit 5 as shown in FIG. 2. Further, when the hand piece 1 is used, the transducer unit 2 and the probe unit 3 are coupled to each other. As a result of this, in the connected body of the transducer unit 2 and the probe unit 3, a first high-frequency electric pathway 13 through which a high-frequency current is transmitted is formed.

Subsequently, the handle unit 4 and the sheath unit 5 are coupled to each other. When the handle unit 4 and the sheath unit 5 are coupled to each other, in a state where the knob member 32 of the sheath unit 5 is grasped, the connection tubular body 34 is inserted into the inside of the rotation transmission member 71 of the handle unit 4. When this sheath unit 5 and the handle unit 4 are coupled to each other, the engagement lever 43 of the handle unit 4 side is held in a state where the engagement lever 43 runs on the inclined surface of the guide groove of the knob member 32 of the sheath unit 5. At this time, as shown in FIG. 22A, the engagement lever 43 is held at a position at which the shape of the inner circumferential surface of the conductive rubber ring 94b and the shape of engagement section 46 of the connection flange section 33c correspond to each other, i.e., in a state where the three corner sections 46b of the connection flange section 33c and the three corner sections 94b2 of the conductive rubber ring 94b coincide with each other. Accordingly, the connection flange section 33c of the sheath unit 5 is inserted straight in the inside of the conductive rubber ring 94b. At this insertion operation time, the conductive rubber ring 94b is held at a non-compression position in the natural state as shown in FIG. 22A. In this state, the sheath unit side electric pathway 40 and the handle unit side electric pathway 95 are not electrically connected to each other.

Subsequently, after this insertion operation is completed, an operation of rotating the knob member 32 of the sheath unit 5 around the axis with respect to the handle unit 4 is performed. By this operation, the engagement lever 43 of handle unit 4 side is engaged with the engagement concave section 42 at one end part of the guide groove in a state where the lever 43 is inserted in the concave section 42. At this time, as shown in FIG. 22B, the position of the conductive rubber ring 94b is switched to the compression contact position at which the rubber ring 94b is brought into compression contact with the three corner sections 46b of the connection flange section 33c. As a result of this, the sheath unit side electric pathway 40 and the handle unit side electric pathway 95 are connected to each other through the conductive rubber ring 94b. As a result of this, in the connected body of the sheath unit 5 and the handle unit 4, a second high-frequency electric pathway 97 through which a high-frequency current is transmitted is formed.

When the sheath unit 5 is operated to be rotated around the axis thereof, the pair of engagement pins 45 of the handle unit 4 side are, at the same time, disengeably engaged with the engagement groove 44a at the termination end of the guide groove 44 of the sheath unit 5. As a result of this, the slider receiving member 64 of the handle unit 4 side and the connection tubular body 34 of the sheath unit 5 side are coupled to each other through the engagement pins 45. As a result of this, it becomes possible for the operation force of the handle unit 4 side at the time when the movable handle 49 is operated to be closed with respect to the fixed handle 47 to be transmitted to the drive pipe 19 of the jaw 17 of the sheath unit 5 side. This state is the state where the sheath unit 5 and the handle unit 4 are coupled to each other.

Thereafter, the connected body of the sheath unit 5 and the handle unit 4, and the connected body of the ultrasonic transducer 6 and the probe unit 3 are assembled into one combined body. At the time of this assembling work, a contact point unit 66 of the handle unit 4 and the front end part of the transducer unit 2 are connected to each other. As a result of this, the second high-frequency electric pathway 97 of the connected body of the sheath unit 5 and the handle unit 4 is connected to the wiring 104 for high-frequency waves transmission inside the cable 9. Further, three wiring lines 105, 106, and 107 inside the cable 9 and a wiring circuit board inside the switch holding section 51 are connected to each other. This state is the completed state of the assembling work of the hand piece 1.

Further, when this hand piece 1 is used, the movable handle 49 is operated to be opened or closed with respect to the fixed handle 47. The drive pipe 19 is moved in the axial direction concomitantly with the operation of the movable handle 49, and the jaw 17 is driven to be opened or closed with respect to the probe distal end section 3a of the probe unit 3 concomitantly with the advancing/retreating operation of the drive pipe 19 in the axial direction. Here, when the movable handle 49 is operated to be closed with respect to the fixed handle 47, the drive pipe 19 is operated to be forwardly pushed concomitantly with the operation of the movable handle 49. The jaw 17 is driven in a direction in which the jaw 17 is made closer to the probe distal end section 3a side of the probe unit 3 (closed position) concomitantly with the pushing operation of the drive pipe 19. When the jaw 17 is operated to be turned to the closed position, the living tissue is grasped between the jaw 17 and the probe distal end section 3a of the probe unit 3.

At this time, in this embodiment, as shown in FIG. 27, at a point of time at which the stroke S of the slider member 65 has changed to S1, and the length of the coil spring 67 has changed to L2, the pressure contact sections 803e of the leaf spring member 803 get over the step section 802c. Thus, the pressure contact sections 803e of the leaf spring member 803 knock the circumferential wall surface of the first cylindrical section 802a on the lower side of the step section 802c, whereby the notification mechanism 801 generates a knocking sound. This makes it possible to notify the user of the appropriate operation amount of the movable handle 49 by a sound.

In this state, either one of the first switch 54 and the second switch 55 of the fixed handle 47 is selectively operated to be depressed. When the first switch 54 is operated to be depressed, a drive current is supplied to the ultrasonic transducer 6 simultaneously with the application of the high-frequency waves, and the ultrasonic transducer 6 is driven. At this time, the ultrasonic vibration from the ultrasonic transducer 6 is transmitted to the probe distal end section 3a through the vibration transmission member 11. As a result of this, it is possible to perform a treatment such as incision, resection, and the like of the living tissue by utilizing the ultrasonic waves together with the application of the high-frequency waves. Incidentally, it is also possible to perform a coagulation treatment of the living tissue by utilizing the ultrasonic waves.

When the second switch 55 is operated to be depressed, each of the first high-frequency electric pathway 13 through which a high-frequency current is transmitted to the probe distal end section 3a of the probe unit 3, and the second high-frequency electric pathway 97 through which a high-frequency current is transmitted to the jaw main body 28 of the sheath unit 5 is turned on. As a result of this, two bipolar electrodes for the high-frequency treatment are constituted by the probe distal end section 3a of the probe unit 3, and the jaw main body 28 of the sheath unit 5. By causing a high-frequency current to flow between the two bipolar electrodes of the probe distal end section 3a of the probe unit 3, and the jaw main body 28 of the sheath unit 5, it is possible to subject the living tissue between the jaw 17 and the probe distal end section 3a of the probe unit 3 to a high-frequency treatment by the bipolar.

Further, when the movable handle 49 is operated to be opened with respect to the fixed handle 47, the drive pipe 19 is operated to be pulled toward the hand side concomitantly with the opening operation of the movable handle 49. The jaw 17 is driven in the direction in which the jaw 17 is separated from the probe distal end section 3a (opened position) concomitantly with the pulling operation of the drive pipe 19.

Further, when the rotary operation knob 50 is operated to be rotated, the rotating operation of the rotation transmission member 71 rotating together with the rotary operation knob 50 is transmitted to the slider receiving member 64 side through the pin 81. As a result of this, when the rotary operation knob 50 is operated to be rotated, the assembled unit of the rotation transmission member 71, pin 81, slider receiving member 64, slider member 65, and coil spring 67 inside the retaining cylinder 48 is driven to be rotated as one body in the direction around the axis together with the rotary operation knob 50. Furthermore, the rotation operation force of the rotary operation knob 50 is transmitted to the vibration transmission member 11 of the probe unit 3 through the tubular member 98 rotating together with the slider receiving member 64 inside the retaining cylinder 48. This allows the assembled unit inside the retaining cylinder 48, together with the connected body of the transducer unit 2 and the probe unit 3 to be rotation-driven as one body in the direction around the axis.

At this time, the knob member 32 of the sheath unit 5, and the guide cylindrical body 33 are rotated together with the rotary operation knob 50. Further, the sheath 18 is rotated together with the guide cylindrical body 33, and the rotation of the guide cylindrical body 33 is transmitted to the connection tubular body 34 and the drive pipe 19 through the rotation restraining pin 235. Accordingly, the jaw 17 of the treatment section 1A, and the probe distal end section 3a are simultaneously driven to be rotated in the direction around the axis together with the rotary operation knob 50.

Thus, the apparatus having the configuration described above produces the following effects. That is, the hand piece 1 of this embodiment is provided with the frictional force reduction section 821 at the contact part at which the inner wall surface of the engagement groove 65a of the slider member 65 and the action pin 58 of the movable handle 49 are in contact with each other. This frictional force reduction section 821 includes a ring-shaped contact member 804 formed of a slippery resin member, for example, a PTFE (polytetrafluoroethylene) resin material such as Teflon (registered trade name), and fixed to the inner wall surface of the engagement groove 65a of the slider member 65. This contact member 804 is arranged on the front side of the action pin 58 of the movable handle 49 inserted in the engagement groove 65a. Thus, when the movable handle 49 is operated in a direction in which the handle 49 is closed with respect to the fixed handle 47, the pressing force applied from the action pin 58 to the wall surface on the front side of the engagement groove 65a can be received by the contact member 804. As a result of this, the action pin 58 can be prevented from being brought into direct contact with the metal surface of the wall surface on the front side of the engagement groove 65a. This makes it possible, when the movable handle 49 is operated in the direction in which the handle 49 is closed with respect to the fixed handle 47, to make the frictional force acting between the action pin 58 and the wall surface on the front side of the engagement groove 65a small. Accordingly, even when the movable handle 49 is operated in the direction in which the handle 49 is closed with respect to the fixed handle 47, the rotary operation knob 50 can be operated to be rotated by slight force. As a result of this, the load of the operation for rotating the rotary operation knob 50 can be prevented from becoming heavy, and a stable operation can be performed.

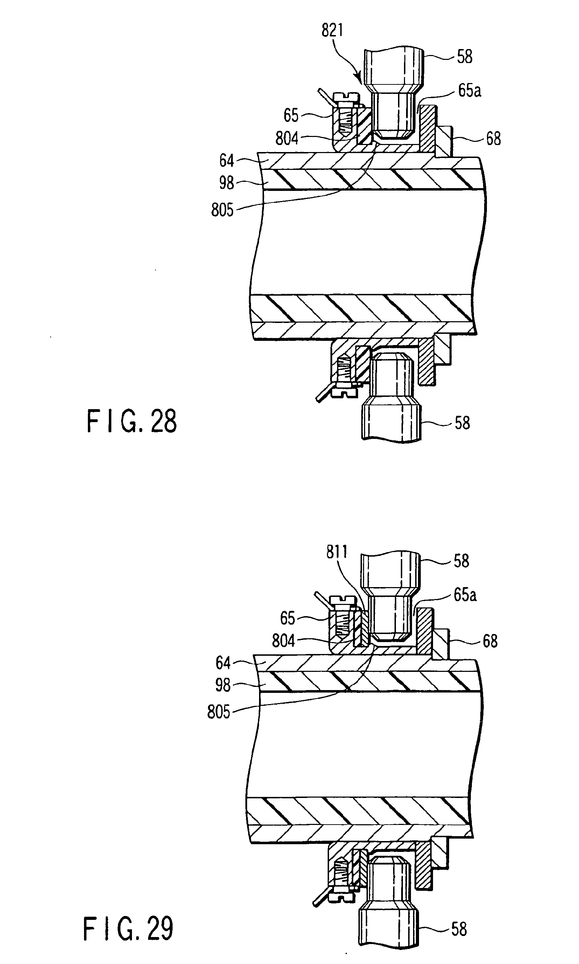

FIG. 29 shows a second embodiment of the present invention. In this embodiment, the configuration of the frictional force reduction section 821 of the first embodiment is changed in the following manner. That is, in this embodiment, a metallic washer 811 for preventing the elasticity of the resin material from being fatigued is provided at an engagement surface at which a contact member 804 and each of pins 58 are in contact with each other.

Thus, the apparatus having the configuration described above produces the following effects. That is, in a hand piece 1 of this embodiment, when a movable handle 49 is operated in the direction in which the handle 49 is closed with respect to a fixed handle 47, the pressing force applied from the action pin 58 to a wall surface on the front side of an engagement groove 65a can be transmitted to a metal surface of a wall surface on the front side of the engagement groove 65a through the metallic washer 811 and the contact member 804. Accordingly, the action pin 58 can be prevented from being brought into direct contact with the metal surface of the wall surface on the front side of the engagement groove 65a. This makes it possible, when the movable handle 49 is operated in the direction in which the handle 49 is closed with respect to the fixed handle 47, to make the frictional force acting between the action pin 58 and the wall surface on the front side of the engagement groove 65a small. Accordingly, even when the movable handle 49 is operated in the direction in which the handle 49 is closed with respect to the fixed handle 47, a rotary operation knob 50 can be operated to be rotated by slight force. As a result of this, the load of the operation for rotating the rotary operation knob 50 can be prevented from becoming heavy, and a stable operation can be performed.

Furthermore, in this embodiment, the metallic washer 811 for preventing the elasticity of the resin material from being fatigued is provided at the engagement surface at which the contact member 804 and each of the pins 58 are in contact with each other. Here, the engagement surface at which the contact member 804 and each the pins 58 are in contact with each other is only a part of the entire ring-shaped contact member 804. Further, the part of the contact member 804, i.e., the engagement surface at which the contact member 804 and each of the pins 58 are in contact with each other receives the large pressing force from the pin 58. Accordingly, when the metallic washer 811 is not provided, a part of the ring-shaped contact member 804 which is relatively soft locally receives the pressing force from each of the pins 58, and hence at the part of the contact member 804 at which the contact member 804 is brought into contact with each of the pins 58, fatigue of the elasticity of the resin material is more liable to occur than at the other part. Conversely, as in this embodiment, by providing the metallic washer 811 at the engagement surface at which the contact member 804 and each of the pins 58 are in contact with each other, the pressing force from the pins 58 can be received in a state where the pressing force is dispersed to the entirety of the washer 811. Thus, it is possible to prevent the elasticity of the resin material from being locally fatigued by the pressing force from the pins 58 at a part of the contact member 804.

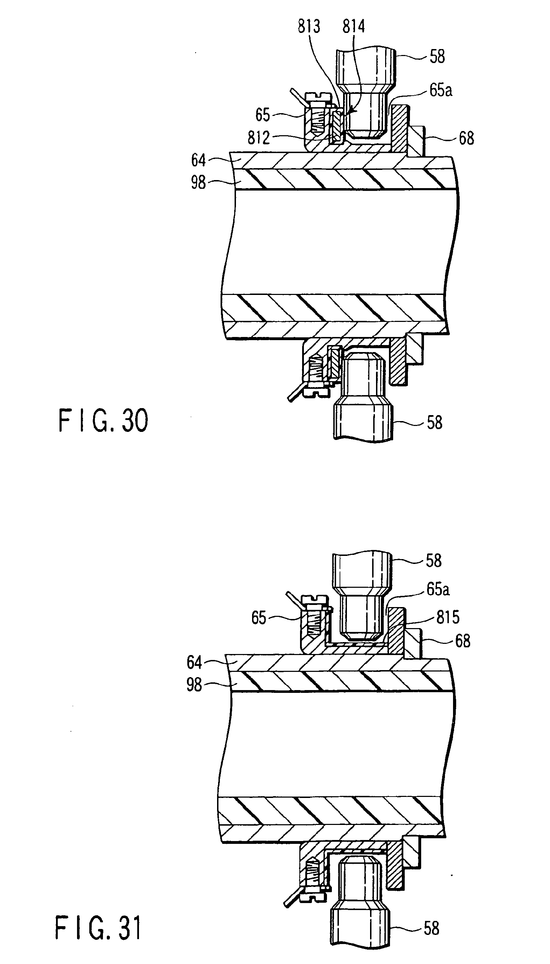

FIG. 30 shows a third embodiment of the present invention. In this embodiment, the configuration of the frictional force reduction section 821 of the first embodiment is changed in the following manner. That is, in this embodiment, in place of the contact member 804, a ring-shaped contact member 814 provided with a coating section 813 formed by coating the surface of a metallic washer 812 with a resin material having low frictional properties is provided.

Thus, the apparatus having the configuration described above produces the following effects. That is, in a hand piece 1 of this embodiment, when a movable handle 49 is operated in the direction in which the handle 49 is closed with respect to a fixed handle 47, the pressing force applied from an action pin 58 to a wall surface on the front side of an engagement groove 65a can be transmitted to a metal surface of the wall surface on the front side of the engagement groove 65a through the ring-shaped contact member 814. Accordingly, the action pin 58 can be prevented from being brought into direct contact with the metal surface of the wall surface on the front side of the engagement groove 65a. This makes it possible, when the movable handle 49 is operated in the direction in which the handle 49 is closed with respect to the fixed handle 47, to make the frictional force acting between the action pin 58 and the wall surface on the front side of the engagement groove 65a small. Accordingly, even when the movable handle 49 is operated in the direction in which the handle 49 is closed with respect to the fixed handle 47, a rotary operation knob 50 can be operated to be rotated by slight force. As a result of this, the load of the operation for rotating the rotary operation knob 50 can be prevented from becoming heavy, and a stable operation can be performed.

FIG. 31 shows a fourth embodiment of the present invention. In this embodiment, the configuration of the frictional force reduction section 821 of the first embodiment is changed in the following manner. That is, in this embodiment, on an inner wall surface of an engagement groove 65a of a slider member 65, a coating section 815 coated with a resin material having low frictional properties is provided.

Thus, the apparatus having the configuration described above produces the following effects. That is, in a hand piece 1 of this embodiment, when a movable handle 49 is operated in the direction in which the handle 49 is closed with respect to a fixed handle 47, the pressing force applied from an action pin 58 to a wall surface on the front side of the engagement groove 65a can be transmitted to a metal surface of the wall surface on the front side of the engagement groove 65a through the coating section 815 of the inner wall surface of the engagement groove 65a. Accordingly, the action pin 58 can be prevented from being brought into direct contact with the metal surface of the wall surface on the front side of the engagement groove 65a. This makes it possible, when the movable handle 49 is operated in the direction in which the handle 49 is closed with respect to the fixed handle 47, to make the frictional force acting between the action pin 58 and the wall surface on the front side of the engagement groove 65a small. Accordingly, even when the movable handle 49 is operated in the direction in which the handle 49 is closed with respect to the fixed handle 47, a rotary operation knob 50 can be operated to be rotated by slight force. As a result of this, the load of the operation for rotating the rotary operation knob 50 can be prevented from becoming heavy, and a stable operation can be performed.

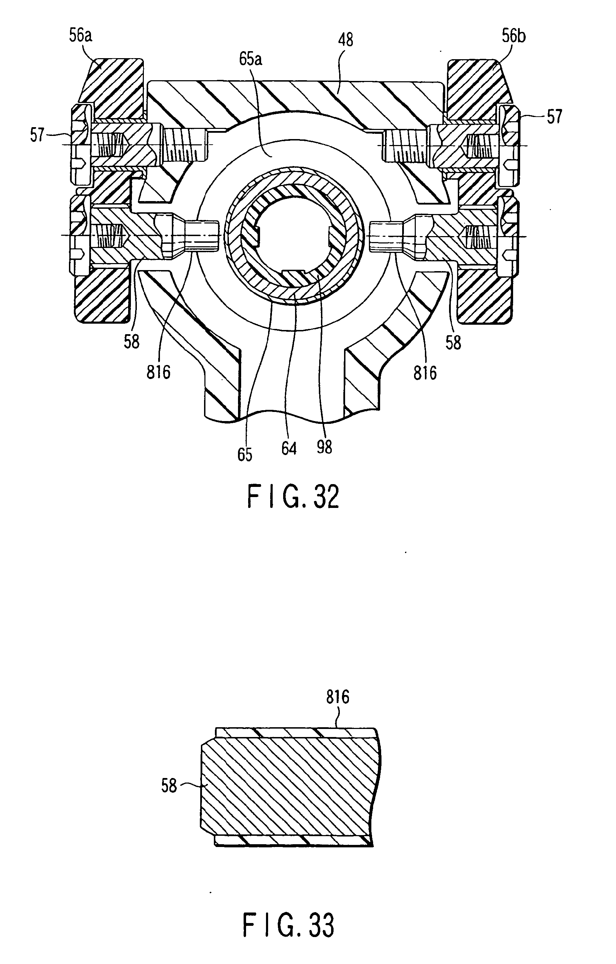

FIGS. 32 and 33 show a fifth embodiment of the present invention. In this embodiment, the configuration of the frictional force reduction section 821 of the first embodiment is changed in the following manner. That is, in this embodiment, a coating section 816 formed by coating each of actions pins 58 of a movable handle 49 with a resin material having low frictional properties is provided on a surface of each of the action pins 58.

Thus, the apparatus having the configuration described above produces the following effects. That is, in a hand piece 1 of this embodiment, when a movable handle 49 is operated in the direction in which the handle 49 is closed with respect to a fixed handle 47, the pressing force applied from an action pin 58 to a wall surface on the front side of an engagement groove 65a can be transmitted to a metal surface of the wall surface on the front side of the engagement groove 65a through the coating section 816 of each of the action pins 58. Accordingly, the metallic part of the action pin 58 can be prevented from being brought into direct contact with the metal surface of the wall surface on the front side of the engagement groove 65a. This makes it possible, when the movable handle 49 is operated in the direction in which the handle 49 is closed with respect to the fixed handle 47, to make the frictional force acting between the action pin 58 and the wall surface on the front side of the engagement groove 65a small. Accordingly, even when the movable handle 49 is operated in the direction in which the handle 49 is closed with respect to the fixed handle 47, a rotary operation knob 50 can be operated to be rotated by slight force. As a result of this, the load of the operation for rotating the rotary operation knob 50 can be prevented from becoming heavy, and a stable operation can be performed.

Incidentally, the present invention is not limited to the embodiments described above. For example, in the fifth embodiment, the coating section 816 formed by coating the action pin 58 of the movable handle 49 with the resin material having low frictional properties is provided on the surface of the action pin 58. Instead of providing the coating section 816, the action pin 58 itself may be formed of a resin member having low frictional properties. Further, any one of the first to fourth embodiments may be combined with the fifth embodiment. Needless to say, furthermore, the present invention may be variously modified to be implemented within the scope of the claims.

## Claims

1. A surgical operating apparatus **characterized by** comprising:
a sheath (18) provided with a distal end part and a proximal end part;
an apparatus main body to be coupled to the proximal end part of the sheath (18);
a probe (11) which is inserted into the sheath (18), and transmits ultrasonic waves;
a probe distal end section (3a) provided at a distal end part of the probe (11); and
a jaw (17) pivotally supported at the distal end part of the sheath (18), the jaw (17) being supported so that the jaw (17) can be operated to be opened or closed between a closed position at which the jaw (17) is engaged with the probe distal end section (3a), and an opened position at which the jaw (17) is separated from the probe distal end section (3a), wherein
the apparatus main body includes:
a handle (49) for operating the opening/closing operation of the jaw (17);
a slider section (65) which is moved to be advanced/retreated in an axial direction of a central axis line of the probe (11) in accordance with the operation of the handle (49);
a pin (58) which is provided to the handle (49), is engaged with the slider section (65), and transmits the operation of the handle (49) to the slider section (65); and **characterised in that**
a frictional force reduction section (821) is provided to the slider section (65) and/or the pin (58) at a surface at which the slider section (65) and the pin (58) are engaged with each other, and reduces the frictional force acting at the engagement surface at which the slider section (65) and the pin (58) are engaged with each other.

2. The surgical operating apparatus according to claim 1, **characterized in that**
the frictional force reduction section (821) is a resin member with low frictional properties.

3. The surgical operating apparatus according to claim 2, **characterized in that**
the resin member with low frictional properties is formed of a PTFE (polytetrafluoroethylene) resin material.

4. The surgical operating apparatus according to claim 2, **characterized in that**
the frictional force reduction section (821) is a ring-shaped contact member (804) which is provided at the engagement surface of the slider section (65) at which the slider section (65) and the pin (58) are engaged with each other, and is formed of the resin member with low frictional properties.

5. The surgical operating apparatus according to claim 4, **characterized in that**
the slider section (65) is provided with a fixing projection (805) for fixing the contact member (804) to the engagement surface of the slider section (65) at which the slider section (65) and the pin (58) are engaged with each other.

6. The surgical operating apparatus according to claim 4, **characterized in that**
the frictional force reduction section (821) is provided with a metallic washer (811) for preventing the elasticity of the resin material of the contact member (804) from being fatigued at an engagement surface at which the contact member (804) and the pin (58) are engaged with each other.

7. The surgical operating apparatus according to claim 2, **characterized in that**
the frictional force reduction section (821) is a coating section (813) which is provided at the engagement surface of the slider section (65) at which the slider section (65) and the pin (58) are engaged with each other, and is formed by coating the surface of a metallic washer with the resin member with low frictional properties.

8. The surgical operating apparatus according to claim 2, **characterized in that**
the frictional force reduction section (821) is a slider section (65) formed of the resin material (815) with low frictional properties.

9. The surgical operating apparatus according to claim 2, **characterized in that**
the frictional force reduction section (821) is the pin (58) formed of the resin material with low frictional properties.

10. The surgical operating apparatus according to claim 2, **characterized in that**
the frictional force reduction section (821) is a coating section (816) formed by coating the surface of the metallic pin (58) with the resin material with low frictional properties.

## Patentansprüche

1. Chirurgisches Operationsinstrument, das **dadurch gekennzeichnet ist, dass** es umfasst:
- eine Hülse (18), die mit einem distalen Endstück und einem proximalen Endstück versehen ist,
- einen Instrumentenhauptkörper zur Verbindung mit dem proximalen Endstück der Hülse (18),
- eine Sonde (11), die in die Hülse (18) eingesetzt wird und Ultraschallwellen aussendet,
- einen distalen Sondenendabschnitt (3a), der an einem distalen Endstück der Sonde (11) bereitgestellt ist,
- eine Klaue (17), die am distalen Endstück der Hülse (18) schwenkbar gehalten wird, wobei die Klaue (17) so gehalten wird, dass die Klaue (17) betätigt werden kann, um zwischen einer geschlossenen Stellung, in der die Klaue (17) mit dem distalen Sondenendabschnitt (3a) in Eingriff steht, und einer offenen Stellung, in der die Klaue (17) vom distalen Sondenendabschnitt (3a) getrennt ist, geöffnet oder geschlossen zu werden, wobei
- der Instrumentenhauptkörper umfasst:
- einen Griff (49) zum Ausführen des Öffnungs-/Schließbetriebs der Klaue (17),
- einen Schieberabschnitt (65), der bewegt wird, um in axialer Richtung einer Mittelachsenlinie der Sonde (11) gemäß der Betätigung des Griffs (49) vorgeschoben bzw. zurückgezogen zu werden,
- einen an dem Griff (49) vorgesehenen Stift (58), der mit dem Schieberabschnitt (65) in Eingriff steht und die Betätigung des Griffs
- (49) auf den Schieberabschnitt (65) überträgt, und
**dadurch gekennzeichnet, dass**
- ein Reibungskraftverringerungsabschnitt (821) an dem Schieberabschnitt (65) und/oder an dem Stift (58) an einer Oberfläche vorgesehen ist, an der der Schieberabschnitt (65) und der Stift (58) miteinander in Eingriff stehen, und die Reibungskraft verringert, die an der Eingriffsfläche wirkt, an der der Schieberabschnitt (65) und der Stift (58) miteinander in Eingriff stehen.

2. Chirurgisches Operationsinstrument nach Anspruch 1,
**dadurch gekennzeichnet, dass** der Reibungskraftverringerungsabschnitt (821) ein Harzteil mit Niedrigreibungseigenschaften ist.

3. Chirurgisches Operationsinstrument nach Anspruch 2,
**dadurch gekennzeichnet, dass** das Harzteil mit Niedrigreibungseigenschaften aus einem PTFE- (Polytetrafluorethylen-) Harzmaterial gebildet ist.

4. Chirurgisches Operationsinstrument nach Anspruch 2,
**dadurch gekennzeichnet, dass** der Reibungskraftverringerungsabschnitt (821) ein ringförmiges Kontaktelement (804) ist, das an der Eingriffsfläche des Schieberabschnitts (65) bereitgestellt ist, an der der Schieberabschnitt (65) und der Stift (58) miteinander in Eingriff stehen, und aus dem Harzteil mit Niedrigreibungseigenschaften gebildet ist.

5. Chirurgisches Operationsinstrument nach Anspruch 4,
**dadurch gekennzeichnet, dass** der Schieberabschnitt (65) mit einem Fixierungsvorsprung (805) versehen ist, um das Kontaktelement (804) an der Eingriffsfläche des Schieberabschnitts (65) zu fixieren, an der der Schieberabschnitt (65) und der Stift (58) miteinander in Eingriff stehen.

6. Chirurgisches Operationsinstrument nach Anspruch 4,
**dadurch gekennzeichnet, dass** der Reibungskraftverringerungsabschnitt (821) mit einer metallischen Beilagscheibe (811) versehen ist, um zu verhindern, dass die Elastizität des Harzmaterials des Kontaktelements (804) an der Eingriffsfläche ermüdet, an der das Kontaktelement (804) und der Stift (58) miteinander in Eingriff stehen.

7. Chirurgisches Operationsinstrument nach Anspruch 2,
**dadurch gekennzeichnet, dass** der Reibungskraftverringerungsabschnitt (821) ein Beschichtungsabschnitt (813) ist, der an der Eingriffsfläche des Schieberabschnitts (65) vorgesehen ist, an der der Schieberabschnitt (65) und der Stift (58) miteinander in Eingriff stehen, und durch Beschichten der Oberfläche einer metallischen Beilagscheibe mit dem Harzteil mit Niedrigreibungseigenschaften gebildet wird.

8. Chirurgisches Operationsinstrument nach Anspruch 2,
**dadurch gekennzeichnet, dass** der Reibungskraftverringerungsabschnitt (821) ein Schieberabschnitt (65) ist, der aus dem Harzmaterial (815) mit Niedrigreibungseigenschaften gebildet ist.

9. Chirurgisches Operationsinstrument nach Anspruch 2,
**dadurch gekennzeichnet, dass** der Reibungskraftverringerungsabschnitt (821) der Stift (58) ist, der aus dem Harzmaterial mit Niedrigreibungseigenschaften gebildet ist.

10. Chirurgisches Operationsinstrument nach Anspruch 2,
**dadurch gekennzeichnet, dass** der Reibungskraftverringerungsabschnitt (821) ein Beschichtungsabschnitt (816) ist, der durch Beschichten der Oberfläche des metallischen Stifts (58) mit dem Harzmaterial mit Niedrigreibungseigenschaften gebildet wird.

## Revendications

1. Appareil pour opération chirurgicale, **caractérisé par** le fait de comprendre :
une gaine (18) munie d'une partie d'extrémité distale et d'une partie d'extrémité proximale ;
un corps principal d'appareil, à coupler à la partie d'extrémité proximale de la gaine (18) ;
une sonde (11), insérée dans la gaine (18) et transmettant des ondes ultrasonores ;
une section d'extrémité distale de sonde (3a), prévue à une partie d'extrémité distale de la sonde (11) ; et
une mâchoire (17), supportée à pivotement à la partie d'extrémité distale de la gaine (18), la mâchoire (17) étant supportée de manière que la mâchoire (17) puisse être actionnée pour être ouverte ou fermée, entre une position fermée, à laquelle la mâchoire (17) est en prise avec la section d'extrémité distale de sonde (3a), et une position ouverte, à laquelle la mâchoire (17) est séparée de la section d'extrémité distale de sonde (3a), dans lequel
le corps principal d'appareil comprend :
une poignée (49) pour actionner l'opération d'ouverture/fermeture de la mâchoire (17) ;
une section formant coulisseau (65), déplacée pour être avancée/rétractée dans une direction axiale d'un axe central de la sonde (11), selon l'actionnement de la poignée (49) ;
une tige (58), prévue sur la poignée (49), mise en prise avec la section formant coulisseau (65), et transmettant l'actionnement de la poignée (49) à la section formant coulisseau (65) ; et **caractérisé en ce que**
une section de réduction de force de friction (821) est prévue sur la section formant coulisseau (65) et/ou la tige (58), à une surface à laquelle la section formant coulisseau (65) et la tige (58) sont mises en prise l'une avec l'autre, et réduit la force de friction agissant au niveau de la surface de contact à laquelle la section formant coulisseau (65) et la tige (58) sont mise en contact l'une avec l'autre.

2. Appareil pour opération chirurgicale selon la revendication 1, **caractérisé en ce que**
la section de réduction de force de friction (821) est un organe en résine, ayant des propriétés de faible friction.

3. Appareil pour opération chirurgicale selon la revendication 2, **caractérisé en ce que**
l'organe en résine, ayant des propriétés de faible friction est formé de matériau de résine PTFE (polytétrafluoroéthylène).

4. Appareil pour opération chirurgicale selon la revendication 21, **caractérisé en ce que**
la section de réduction de force de friction (821) est un organe de contact (804) en forme d'anneau, prévu sur la surface de contact de la section formant coulisseau (65) à laquelle la section formant coulisseau (65) et la tige (58) sont en contact l'une avec l'autre, et est formé de l'organe en résine ayant des propriétés de faible friction.

5. Appareil pour opération chirurgicale selon la revendication 4, **caractérisé en ce que**
la section formant coulisseau (65) est munie d'une saillie de fixation (05), pour fixer l'organe de contact (804) à la surface de contact de la section formant coulisseau (65) à laquelle la section formant coulisseau (65) et la tige (58) sont en contact l'une avec l'autre.

6. Appareil pour opération chirurgicale selon la revendication 4, **caractérisé en ce que**
la section de réduction de force de friction (821) est munie d'une rondelle (811) métallique, pour empêcher que l'élasticité du matériau de résine de l'organe de contact (804) ne soit fatigué à une surface de contact à laquelle l'organe de contact (804) et la tige (58) sont en contact l'une avec l'autre.

7. Appareil pour opération chirurgicale selon la revendication 2, **caractérisé en ce que**
la section de réduction de force de friction (821) est une section à revêtement (813), prévue à la surface de contact de la section formant coulisseau (65) à laquelle la section formant coulisseau (65) et la tige (58) sont en contact l'une avec l'autre, et est formée par revêtement de la surface d'une rondelle métallique avec l'organe en résine ayant des propriétés de faible friction.

8. Appareil pour opération chirurgicale selon la revendication 2, **caractérisé en ce que**
la section de réduction de force de friction (821) est une section formant coulisseau (65), formée du matériau de résine ayant des propriétés de faible friction.

9. Appareil pour opération chirurgicale selon la revendication 2, **caractérisé en ce que**
la section de réduction de force de friction (821) est la tige (58), formée du matériau de résine ayant des propriétés de faible friction.

10. Appareil pour opération chirurgicale selon la revendication 2, **caractérisé en ce que**
la section de réduction de force de friction (821) est une section à revêtement (816) formée par revêtement de la surface de la tige métallique (58) avec le matériau de résine ayant des propriétés de faible friction.
